# EUROPÄISCHE PATENTSCHRIFT

(11) **EP 1 017 723 B1**
(45) Veröffentlichungstag und Bekanntmachung des Hinweises auf die Patenterteilung: **10.01.2007**
(21) Anmeldenummer: 98956800.1
(22) Anmeldetag: 23.09.1998
(51) Int. Cl.: C07K 14/705, C12N 15/12, C12N 5/10, C07K 16/28, C12N 5/12, G01N 33/53, A61K 38/17, C12Q 1/68

(54) **KO-STIMULIERENDES POLYPEPTID VON T-ZELLEN, MONOKLONALE ANTIKÖRPER SOWIE DIE HERSTELLUNG UND DEREN VERWENDUNG**
COSTIMULATING T-CELL POLYPEPTIDE, MONOCLONAL ANTIBODIES, THEIR PREPARATION AND USE
POLYPEPTIDE COSTIMULANT DE LYMPHOCYTES T, ANTICORPS MONOCLONAUX, LEUR PRODUCTION ET LEUR UTILISATION

(30) Priorität: 23.09.1997 DE 19741929; 11.05.1998 DE 19821060
(43) Veröffentlichungstag der Anmeldung: 12.07.2000
(73) Patentinhaber: Bundesrepublik Deutschland letztvertreten durch Den Direktor des Robert-Koch-Instituts, 13353 Berlin (DE)
(72) Erfinder: KROCZEK, Richard, Nordufer 20 D-13353 Berlin (DE)
(74) Vertreter: Bösl, Raphael Konrad
(86) Internationale Anmeldenummer: PCT/DE1998/002896
(87) Internationale Veröffentlichungsnummer: WO 1999/015553

(56) Entgegenhaltungen:
- EP-A- 0 984 023
- WO-A-89/10398
- WO-A-90/04180
- LANIER L L ET AL: "CD80 (B7) AND CD86 (B70) PROVIDE SIMILAR COSTIMULATORY SIGNALS FOR T CELL PROLIFERATION, CYTOKINE PRODUCTION, AND GENERATION OF CTL" JOURNAL OF IMMUNOLOGY, Bd. 154, 1. Januar 1995, Seiten 97-105, XP002018932
- FREEMAN G J ET AL: "CLONING OF B-2: A CTLA-4 COUNTER-RECEPTOR THAT COSTIMULATES HUMAN T CELL PROLIFERATION" SCIENCE, Bd. 262, 5. November 1993, Seiten 909-911, XP002069402
- TAMATANI T, TEZUKA K: "Human Cell Surface Protein " GENESEQ DATABASE, ACCESSION NUMBER W75956, XP002094461 & WO 98 38216 A (JAPAN TOBACCO INC) 3. September 1998
- TAMATANI T, TEZUKA K: "Human cell surface protein encoding cDNA" GENESEQ DATABASE, ACCESSION NUMBER V53199, XP002094462 & WO 98 38216 A (JAPAN TOBACCO INC) 3. September 1998
- REDOGLIA V, ET AL.: "Characterization of H4: a mouse T lymphocyte activation molecule functionally associated with the CD3/T cell recepto" EUR. J. IMMUNOL., Bd. 26, 1996, Seiten 2781-2789, XP001013533

## Beschreibung

Die Erfindung betrifft Maus-Antikörper gegen das 8F4-Molekül und Hybridomzellen, die die monoklonalen Antikörper herstellen. Ferner betrifft die Erfindung die Verwendung von monoklonalen Antikörpern, die biologische Aktivität des erfindungsgemäßen Polypeptids 8F4 hemmen als Arzneimittel. Insbesondere betrifft die Erfindung die Verwendung dieser Substanzen zur Vorbeugung oder Therapie von Erkrankungen an denen das Immunsystem beteiligt ist, insbesondere zur Behandlung von Autoimmunkrankheiten und zur Verhinderung der Abstoßungsreaktionen bei Organtransplantationen. Ferner betrifft die Erfindung die Verwendung des 8F4-Moleküls mit der biologischen Aktivität der Ko-Stimulation von T-Zellen oder von Zellen, die das 8F4-Molekül enthalten, als Arzneimittel, insbesondere zur Vorbeugung oder Therapie von Erkrankungen an denen das Immunsystem beteiligt ist, insbesondere zur Behandlung von Krebserkrankungen, Aids, Asthmaerkrankungen oder chronischen viralen Erkrankungen wie HCV- oder HBV-Infektionen. Weiterhin betrifft die Erfindung die Verwendung von monoklonalen Antikörpern zur Diagnose von Erkrankungen, an denen das Immunsystem beteiligt ist Insbesondere betrifft die Erfindung die Diagnose mittels eines ELISA-Nachweises, einer Durchflußzytometrie, eines Western Blot, eines Radioimmunnachweises, einer Nephelometrie oder einer histochemischen Anfärbung.

T-Lymphozyten erkennen ihr Antigen, das von "Antigen-präsentierenden Zellen", z.B. dendritischen Zellen, B-Zellen und Makrophagen, dargeboten wird, durch ihren T-Zell-Rezeptor. Die Erkennung des Antigens durch den T-Zell-Rezeptor alleine aber reicht in den meisten Fällen nicht aus, um T-Lymphozyten ausreichend zu aktivieren. Hierzu bedarf es der zusätzlichen, gleichzeitigen Stimulation (nachfolgend auch "Ko-Stimulation" genannt) durch andere Rezeptormoleküle auf der Oberfläche der T-Lymphozyten. Eines dieser Rezeptormoleküle ist der sogenannte CD28-Rezeptor, der durch das ko-stimulierende Molekül B7 stimuliert wird. Werden diese "ko-stimulatorischen" Moleküle, z.B. CD28, wirksam, so erreicht die Aktivierung der T-Zelien nach der Erkennung des Antigens durch den T-Zell-Rezeptor ein ausreichendes Niveau. Nach einer solchen vollständigen Aktivierung exprimiert die T-Zelle zusätzliche Moleküle, z.B. CD25, CD69, CD71, auf der Oberfläche und synthetisiert zahlreiche Zytokine, z.B. IL-2 und IFN-γ, welche die Funktion von Botenstoffen haben. Sowohl diese zusätzlichen Oberflächenmoleküle wie auch die Zytokine dienen dem Informationsaustausch der T-Zelle mit anderen Zellen des Immunsystems. Durch die zusätzlichen Oberflächenmoleküle und die Zytokine lenken die aktivierten T-Zellen die gesamte Antigen-spezifische Immunabwehr. Auf diese Weise wird sowohl die Generierung von zytotoxischen Zellen ("Killerzellen") wie auch die Generierung von Antigen-spezifischen Antikörpern durch B-Zellen gesteuert. Zytotoxische Zellen wie auch die spezifisch gebildeten Antikörper eliminieren virale oder bakterielle Erreger, welche in den Körper eindringen. In einigen Fällen kommt es jedoch zu einem Überschießen der Immunreaktion und das Immunsystem richtet sich gegen die eigenen Körperzellen. Das führt zum Auftreten von "Autoimmunerkrankungen", z.B. zu rheumatoider Arthritis, Morbus Bechterew, Sjögren-Syndrom, Colitis ulcerosa, u.a. Einer der wesentlichen Orte der Kooperation zwischen Antigen-aktivierten T-Zellen und anderen Zellen des Immunsystems sind die sekundären lymphatischen Organe, darunter die Tonsillen. Hier werden die T-Lymphozyten durch das von dendritischen Zellen präsentierte Antigen aktiviert, hier interagieren T-Lymphozyten mit B-Zellen. Aufgrund dieser Interaktion sezernieren B-Zellen nach mehreren Zwischenstufen der Differenzierung Antigen-spezifische Antikörper vom IgM- und IgG-Typ.

Das am besten charakterisierte und bisher mit wirksamste ko-stimulatorische Molekül ist das CD28-Oberflächenmolekül (nachstehend CD28-Rezeptor oder CD28 genannt), das auf einem großen Teil der T-Zellen konstitutiv exprimiert wird. *In vitro* führt die Ko-Stimulation durch CD28 nach Erkennung des Antigens durch den T-Zell-Rezeptor zu einer sehr starken Erhöhung der Zytokin-Sekretion von z.B. IL-2 und IFN-γ wie auch zu einer deutlichen Heraufregulation der Expression von Zelloberflächen-Molekülen wie CD25, CD69, CD71, welche für die Interaktion der T-Zellen mit anderen Immunzellen, z.B. B-Lymphozyten, notwendig sind; vgl. Chambers und Allison, *Current Opinion in Immunology* 9 (1997), 396-404. Durch die Ko-Stimulation über den CD28-Rezeptor läßt sich des weiteren die Proliferation der T-Lymphozyten deutlich steigern. Auch wird durch die Ko-Stimulation über den CD28-Rezeptor die T-Zell-Steuerung der B-Lymphozyten-Funktion so optimiert, daß es zu einer erhöhten Sekretion von Antikörpern kommt
Wenn die Funktion des CD28-Rezeptors aufgehoben wird, kommt es zu einer drastischen Funktionseinbuße der Immunabwehr. Das konnte anhand einer transgenen Maus gezeigt werden, in der das CD28-Gen durch homologe Rekombination zerstört wurde (ein sogenannter "CD28-knock-out"). Die auf diese Weise gestörte Aktivierung der Antigen-spezifischen T-Zellen führt zu einer fehlenden Ko-Stimulation. Diese wiederum führt zu einer Störung der T-Zell-Funktion, d.h. zu einer verminderten Proliferation der T-Zellen und zu einer drastisch verminderten Synthese verschiedener Zytokine. Die fehlende Ko-Stimulation führt letztendlich zu einer verminderten Funktion der Antigen-spezifischen Immunabwehr. So wird u.a. durch das Fehlen von CD28 die Bildung Antigen-spezifischer IgG1- und IgG2-Antikörper durch B-Lymphozyten auf 10% des Normwertes reduziert; vgl. Shahinian et al., *Science* 262 (1993), 609-612; Lucas et al. Journal of *Immunology* 154 (1995), 5757-5768.Über eine Ko-Stimulation durch CD28 kann man *in vitro* auch das Eindringen des Aids-Virus in T-Lymphozyten verhindern; vgl. Riley et al., *Journal of Immunology* 158 (1997), 5545-5553. Entsprechende Versuche sind *in vivo* noch nicht durchgeführt worden. Bekanntlich schaltet CD28 viele Zytokin-Gene an, die *in vivo* zu erheblichen Nebenwirkungen führen können. Die Blockade der Rezeptoren von CD28 durch ein lösliches CTLA-4-Immunglobulin-Molekül ist im Affenmodell erfolgreich eingesetzt worden, um die Abstoßung von transplantierten Nieren zu verhindern. Hierbei wurde CTLA-4 in Kombination mit einem Antikörper gegen das CD40-Liganden-Molekül eingesetzt worden; vgl. Kirk et al., *Proc. Natl. Acad. Sci. USA* 94 (1997) 8789-8794. Die Blockade der CD28-Rezeptoren betrifft jedoch sämtliche T-Lymphozyten und nicht nur die bereits aktivierten, da CD28 auf T-Lymphozyten konstitutiv exprimiert wird.

EP 0 984 023 A1 offenbart ein Adhäsionsmolekül, das sowohl auf aktivierten T-Lymphblasten wie auch auf aktivierten B-Lymphoblasten beobachtet wurde. Redoglia V. et al. (1996), Eur. J. Immunol., 26, 2781-2789 offenbaren ein Molekül, das selektiv auf aktivierten Maus-T-Zellen exprimiert und eine Rolle als T-Zell-spezifisches kostimulatorisches Molekül zugeschrieben wird.

Es besteht somit ein Bedarf an einem ko-stimulierenden Oberflächenmolekül, das nur auf humanen aktivierten T-Lymphozyten exprimiert wird. Der Erfindung liegt daher die Aufgabe zugrunde, ein Oberflächenmolekül auf humanen aktivierten T-Zellen, das eine starke ko-stimulatorische Wirkung auf zentrale Funktionen der T-Lymphozyten aufweist, bereitzustellen. Eine weitere Aufgabe der Erfindung ist es, monoklonale Antikörper gegen das ko-stimulatorische Oberflächenmolekül bereitzustellen.

Der Gegenstand der Erfindung wird in den Ansprüchen näher beschrieben.

Die Erfindung offenbart auch ein Polypeptid mit der biologischen Aktivität der Ko-Stimulation von T-Zellen, charakterisiert dadurch, daß a) das Polypeptid auf aktivierten CD4⁺- und CD8⁺-T-Lymphozyten, aber nicht auf ruhenden oder aktivierten B-Zellen, Granulozyten, Monozyten, NK-Zellen (natürliche Killerzellen) oder dendritischen Zellen vorkommt, und b) das Polypeptid ein Dimer ist, wobei das Polypeptid ein Molekulargewicht von etwa 55 bis 60 kDa (Kilodalton) hat, bestimmt in einer nicht reduzierenden Natriumdodecylsulfat-Polyacrylamidgelelektrophorese (SDS-PAGE), und wobei die zwei Polypeptidketten des Polypeptids ein Molekulargewicht von etwa 27 kDa und etwa 29 kDa haben, gemessen in einer reduzierenden SDS-PAGE.

Das Polypeptid (nachstehend auch 8F4-Molekül oder 8F4 genannt) wird erst nach Aktivierung der T-Lymphozyten exprimiert, und zwar sowohl auf CD4⁺- wie auch auf CD8⁺-T-Zellen. In einer nicht reduzierenden SDS-PAGE weist das 8F4-Molekül ein Molekulargewicht zwischen etwa 55 und 60 kDa (Kilodalton) auf. Das 8F4-Molekül ist aus zwei Peptidketten zusammengesetzt, wobei die beiden Peptidketten in einer reduzierenden SDS-PAGE ein Molekulargewicht von etwa 27 und etwa 29 kDa aufweisen. Histologisch kann man das 8F4-Antigen auf aktivierten T-Lymphozyten im lymphatischen Gewebe der Tonsillen und Lymphknoten eindeutig nachweisen, insbesondere in Keimzentren, dem Ort der Interaktion von T-Lymphozyten und B-Lymphozyten bei der Generierung von Antikörpern. *Ex vivo* isolierte tonsilläre T-Zellen sind zu etwa 50-80% positiv für das 8F4-Antigen und weisen Zeichen einer fortgeschrittenen Aktivierung auf. Das 8F4-Molekül ist auf ruhenden oder aktivierten B-Zellen, Granulozyten, Monozyten, NK-Zellen und dendritischen Zellen nicht nachweisbar.

Eine wichtige biologische Aktivität des 8F4-Moleküls ist seine ko-stimulierende Aktivität von T-Lymphozyten. Die ko-stimulierende Aktivität kann bestimmt werden nach Linsley, et al., *Journal of Experimental Medicine* 176 (1992), 1595-604. Die ko-stimulierende Aktivität des 8F4-Moleküls ähnelt der ko-stimulierenden Aktivität des CD28-Moleküls, welches als zentrales Verstärkungselement der Antigen-Erkennung durch das Immunsystem identifiziert worden ist. Das 8F4-Molekül unterscheidet sich jedoch in vielen Aspekten von CD28. So muß die Expression des 8F4-Moleküls auf der Oberfläche der T-Zellen erst induziert werden, während CD28 konstitutiv exprimiert wird. Auch in der Funktion sind deutliche Unterschiede nachweisbar: Die Ko-Stimulation über CD28 führt zur Überexpression zahlreicher Lymphokine, u.a. des Interleukin-2 (IL-2). Auch die Ko-Stimulation über 8F4 führt zu einer verstärkten Sekretion von Lymphokinen, nicht jedoch des IL-2. Somit unterscheidet sich die ko-stimulatorische Aktivität des 8F4-Moleküls von der Aktivität des CD28-Moleküls. Da die Stimulation über 8F4 nicht alle Zytokin-Gene anschaltet, ist eine Ko-Stimulation über 8F4 *in vivo* vorteilhaft, z.B. gegenüber einer Ko-Stimulation über den CD28-Rezeptor. Auch unterscheidet sich die Induktion, die Expression, der Expressionsort und die Funktion des 8F4-Moleküls von allen anderen bekannten ko-stimulatorisch wirksamen Molekülen.

Bei dem 8F4-Molekül handelt es sich um ein Oberflächenmolekül auf aktivierten T-Zellen, das eine starke ko-stimulatorische Wirkung auf zentrale Funktionen der T-Lymphozyten aufweist. Die Expression *in vivo* deutet u.a. auf eine wesentliche Beteiligung des 8F4-Moleküls an der Kooperation von T-Zellen mit anderen Zellen des Immunsystems wie B-Zellen oder dendritischen Zellen im Rahmen der humoralen und zellulären Immunabwehr gegen Viren und Bakterien hin.

Nach Expression hat das 8F4-Molekül *in vitro* eine starke ko-stimulatorische Wirkung auf verschiedene Funktionen der T-Lymphozyten:
1. Deutliche Verstärkung der Proliferation von T-Lymphozyten.
2. Deutliche Verstärkung der Synthese bestimmter Zytokine durch die T-Lymphozyten.
3. Stark erhöhte Expression von Steuerungs-Molekülen, z.B. Oberflächenmoleküle und Zytokine, auf und in T-Lymphozyten.
4. Deutliche Verbesserung der T-Zell-induzierten Antikörper-Bildung (IgM und IgG) durch B-Zellen.

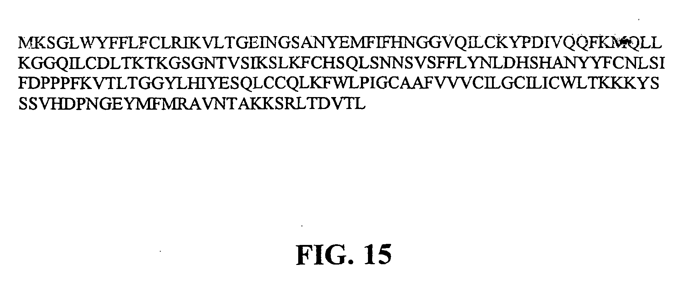

Die vorliegende Erfindung offenbart auch ein Polypeptid mit der biologischen Aktivität der Ko-Stimulation von T-Zellen und mit einer Aminosäuresequenz, die mindestens 40% Homologie mit der 199 Aminosäuren umfassenden Sequenz in Fig. 15 (SEQ ID NO:2) aufweist, oder ein biologisch aktives Fragment oder ein Analogon davon. Ein biologisch aktives Fragment oder Analogon ist ein Fragment oder Analogon, das ebenfalls eine ko-stimulatorische Wirkung auf T-Zellen-Lymphozyten zeigt oder zumindest im Sinne einer Blockade eine biologische Wirkung entfaltet. Bevorzugt ist ein Polypeptid oder ein biologisch aktives Fragment oder Analogon davon, das mindestens 60% Homologie mit der 199 Aminosäuren umfassenden Sequenz in Fig. 15 (SEQ ID NO:2) aufweist. In einer besonders bevorzugten Ausführungsform umfaßt das Polypeptid eine Aminosäuresequenz, die mindestens 80% Homologie mit der 199 Aminosäuren umfassenden Sequenz in Fig. 15 (SEQ ID NO:2) aufweist oder ein biologisch aktives Fragment oder Analogon davon.

Insbesondere ist ein Polypeptid mit der biologischen Aktivität der Ko-Stimulation von T-Zellen und umfassend eine Aminosäuresequenz gemäß Fig. 15 (SEQ ID NO:2), oder ein biologisch aktives Fragment davon offenbart.

Die Erfindung schließt ein allele-Varianten und Fragmente von dem 8F4-Molekül. Diese Varianten schließen ein natürlich vorkommende allele-Varianten, Substitutionsanaloga, in denen eine oder mehrere Aminosäuren substituiert worden sind mit verschiedenen Aminosäuren, Substitutionsanaloga, in denen eine oder mehrere Aminosäuren substituiert worden sind mit verschiedenen Aminosäuren, Deletionsanaloga, in denen eine oder mehrere Aminosäuren deletiert worden sind und Additionsanaloga, bei denen eine oder mehrere Aminosäuren hinzugefügt worden sind. Deletion und Addition von einer oder mehreren Aminosäuren können entweder an einer internen Region des Polypeptids oder an dem Amino- oder Carboxyterminus gemacht werden.

Polypeptide, fusioniert zu hetrologen Polypeptiden, werden ebenfalls umfaßt.

Des weiteren werden DNA-Sequenzen offenbart, die ein erfindungsgemäßes Polypeptid oder ein biologisch aktives Fragment davon kodieren.

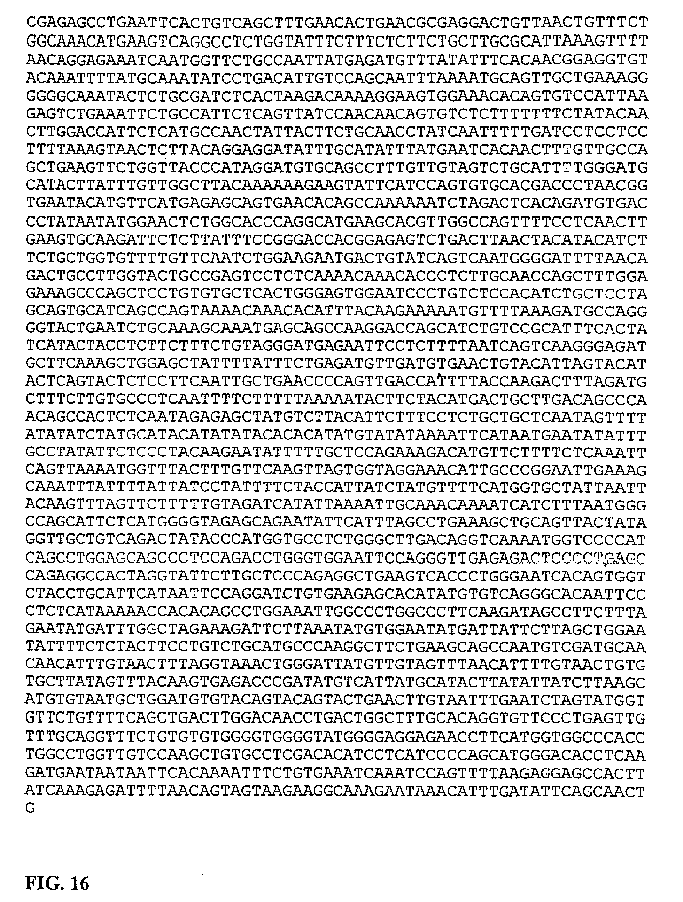

Diese DNA-Sequenzen schließen die Sequenz gemäß SEQ ID NO:1 (Fig. 16) genauso wie allele-Varianten und Fragmente mit biologischer Aktivität ein.

Insbesondere ist eine DNA-Sequenz kodierend ein Polypeptid mit der biologischen Aktivität der Ko-Stimulation von T-Zellen ausgewählt aus der Gruppe bestehend aus:
a) der DNA-Sequenz gemäß SEQ ID NO: 1 (Fig. 16) und ihren komplementären Strang
b) DNA-Sequenz, hybridisierend mit den Sequenzen in (a) und
c) DNA-Sequenzen, die aufgrund der Degeneration des genetischen Codes mit den Sequenzen in (a) und (b) hybridisieren. Vorzugsweise hybridisieren die vorstehenden DNA-Sequenzen zueinander unter stringenten Bedingungen.

Weiterhin werden bereitgestellt Vektoren, die diese DNA-Sequenzen enthalten und Wirtszellen, die mit diesen Vektoren transformiert oder transfiziert sind.

In einer weiteren Ausführungsform offenbart die Erfindung monoklonale Antikörper gegen das 8F4-Molekül. Die monoklonalen Antikörper können auf übliche Weise nach dem von Milstein und Köhler, *Nature* 256 (1975), 495-497, beschriebenen Verfahren hergestellt werden. Insbesondere können die monoklonalen Antikörper hergestellt werden, indem Mäuse mit T-Zellen, die *in vitro* mit Phorbolmyristatacetat (PMA) und Ionomycin ("2-Signal-System") für 24 h aktiviert worden sind, immunisiert werden. Die Milzzellen der immunisierten Mäuse werden mit Myelom-Zellen fusioniert. 8F4-spezifische monoklonale Antikörper werden dadurch identifiziert, daß sie 2-Signal-aktivierte, jedoch nicht ruhende T-Lymphozyten erkennen. Auch färben 8F4-spezifischen Antikörper mit einem Signal (entweder PMA oder lonomycin) stimulierte T-Zellen in einem auf übliche Weise durchgeführten Nachweisverfahren nicht an. 8F4-spezifische Antikörper ergeben ein typisches Anfärbemuster von tonsillären T-Zellen und erkennen ein Antigen von etwa 55 bis 60 kDa in einer nicht reduzierenden SDS-PAGE und von etwa 27 kDa und etwa 29 kDa in einer reduzierenden SDS-PAGE auf aktivierten T-Lymphozyten.

In einer weiteren Ausführungsform betrifft die Erfindung Hybridomzellen, die die erfindungsgemäßen Maus-Antikörper herstellen.

In einer weiteren Ausführungsform betrifft die Erfindung die Verwendung von monoklonalen Antikörpern, die die biologische Aktivität des erfindungsgemäßen Polypeptids 8F4 hemmen als Arzneimittel. Diese Antikörper können als Arzneimittel verwendet werden, zur Vorbeugung oder Therapie von Erkrankungen an denen das Immunsystem beteiligt ist, insbesondere zur Behandlung von Autoimmunkrankheiten oder zur Verhinderung der Abstoßungsreaktionen bei Organtransplantationen. Die Blockade der Interaktion des 8F4-Antigens mit seinem Rezeptor verbessert z.B. die Verhinderung der Organabstoßung, da eine solche Blockade nur bereits aktivierte T-Lymphozyten betrifft. Eine weitere Ausführungsform der Erfindung betrifft die Verwendung des erfindungsgemäßen Polypeptids als Arzneimittel zur Behandlung von Krebserkrankungen, Aids, Asthmaerkrankungen oder chronischen viralen Erkrankungen wie HCV-oder HBV-Infektionen verwendet werden.

Das Polypeptid kann ebenfalls auf übliche Weise in Zellen eingeführt werden, so daß diese Zellen das Polypeptid z.B. konstitutiv exprimieren. Z.B. kann die das Polypeptid codierende Nukleinsäuresequenz oder ein Vektor, umfassend die das Polypeptid codierende Nukleinsäuresequenz, z.B. die cDNA oder genomische DNA, Promotoren, Enhancer und andere für die Expression der Nukleinsäuresequenz benötigten Elemente, in eine Zelle eingeschleust werden. Vorzugsweise wird die 8F4 cDNA (2641 Nukleotide), dargestellt in Fig. 16 (SEQ ID NO: 1), oder Fragmente oder Derivate hiervon zur Expression des Polypeptids oder Fragmenten hiervon eingesetzt.

Ferner kann das Polypeptid z.B. mittels Liposomen in Zellen eingeführt werden, die das Polypeptid danach auf ihrer Zelloberfläche ausbilden. Erfindungsgemäß können diese Zellen als Arzneimittel verwendet werden, insbesondere zur Wiederherstellung der korrekten Regulation des menschlichen Immunsystems, wie sie im Rahmen zahlreicher chronischer Infektionserkrankungen auftritt, z.B. im Rahmen von AIDS, Asthmaerkrankungen oder bei chronischen viralen Hepatitiden (z.B. HCV, HBV), oder zur Stimulation des Immunsystems *in vitro* oder *in vivo,* wie z.B. für die Therapie von Krebserkrankungen verwendet werden.

In einer weiteren Ausführungsform werden die monoklonalen Antikörper zur Diagnose von Erkrankungen, an denen das Immunsystem beteiligt ist verwendet. Zur Diagnose kann z.B. ein ELISA-Nachweis, Durchflußzytometrie, Western Blot, Radioirnmunassay, Nephelometrie oder eine histochemische Anfärbung verwendet werden.

Die monoklonalen Antikörper können den Signaltransduktionsweg des genannten Polypeptids in die T-Zelle positiv oder negativ beeinflussen (modulieren).

Die monoklonalen Antikörper können auch die Heraufregulation des erfindungsgemäßen Polypeptids an die T-Zelloberfläche verhindern.

Des weiteren wird das erfindungsgemäße Polypeptid oder Fragemente hiervon durch ein transgenes Tier exprimiert. Auch ist ein transgenes Tier umfasst, bei dem das Gen, das für das erfindungsgemäße Polypeptid kodiert, ausgeschaltet worden ist ("Knockout")

Die Abbildungen dienen der Erläuterung der Erfindung:
Fig. 1 zeigt das Ergebnis einer Immunpräzipitation des 8F4-Antigens aus aktivierten humanen T-Zellen. (a) Natriumdodecylsulfat-Polyacrylamidgelelektrophorese (SDS-PAGE; 12% Polyacrylamidgel (PAA-Gel)) reduzierend, (b) SDS-PAGE (10% PAA-Gel) nicht reduzierend. Angegeben sind die Bedingungen zur Elution des Antigens von der 8F4-Matrix. "SDS" bedeutet Natriumdodecylsulfat; "DTT" bedeutet Dithiothreitol, "Mr" bedeutet Molekulargewicht und "kDa" bedeutet Kilodalton.

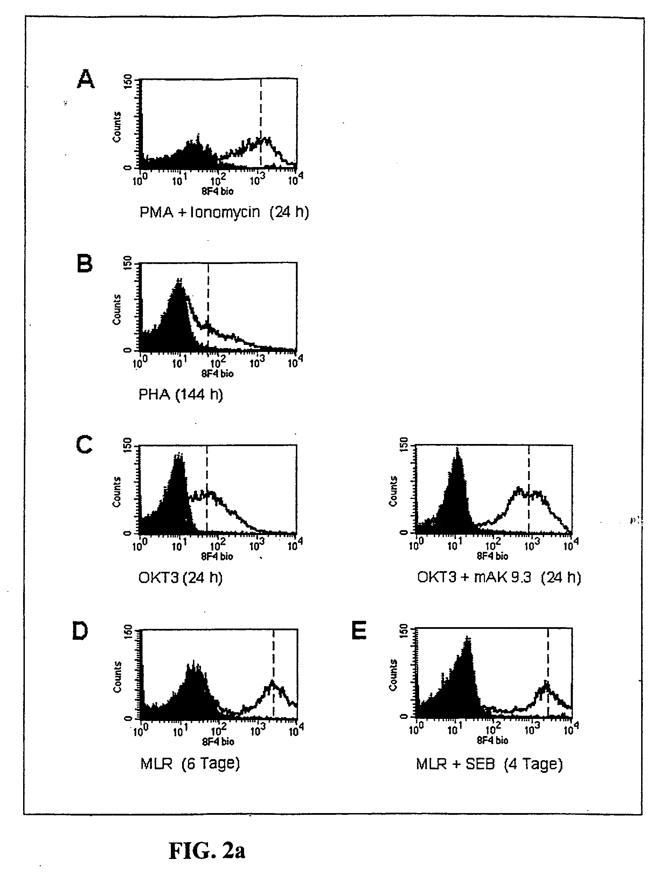

Fig. 2a zeigt das Ergebnis einer Durchflußzytometrie nach Induktion des 8F4-Antigens auf CD4⁺- T-Zellen. Die Aktivierungsdauer der T-Zellen ist in Klammern angegeben. "PMA" bedeutet Phorbolmyristatacetat; "PHA" bedeutet Phytohaemagglutinin; "OKT3" ist ein monoklonaler Antikörper gegen CD3; "MLR" bedeutet gemischte Lymphocytenreaktion (engl.: *mixed lymphocyte reaction*); "mAK 9.3" ist ein monoklonaler Antikörper gegen CD28; "SEB" bedeutet Staphylokokken Enterotoxin B.

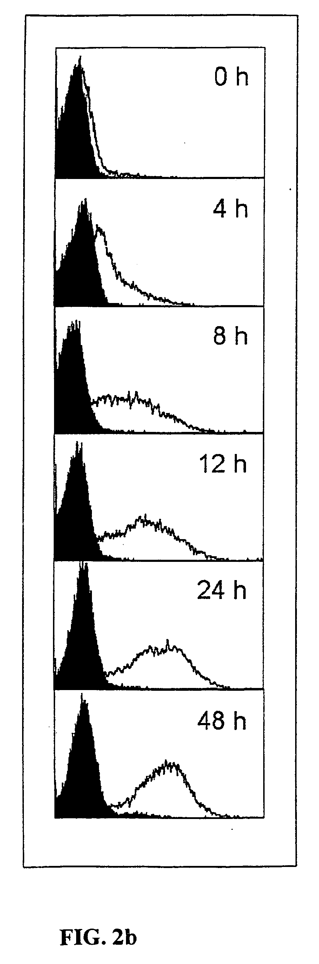

Fig. 2b zeigt das Ergebnis einer Kinetik der Induktion des 8F4-Antigens auf CD4⁺-T-Zellen nach Aktivierung mit PMA und Ionomycin in einer Durchflußzytometrie. Aufgetragen ist die Immunfluoreszenz (log) gegen die Zellzahl.

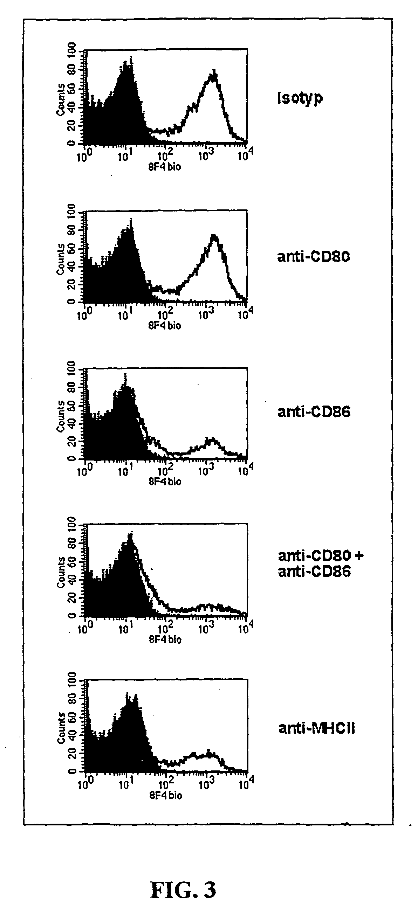

Fig. 3 zeigt das Ergebnis einer Durchflußzytometrie zur Identifikation von Molekülen, welche an der Induktion von 8F4 in der "mixed lymphocyte reaction" beteiligt sind. "bio" bedeutet biotinylierter Antikörper.

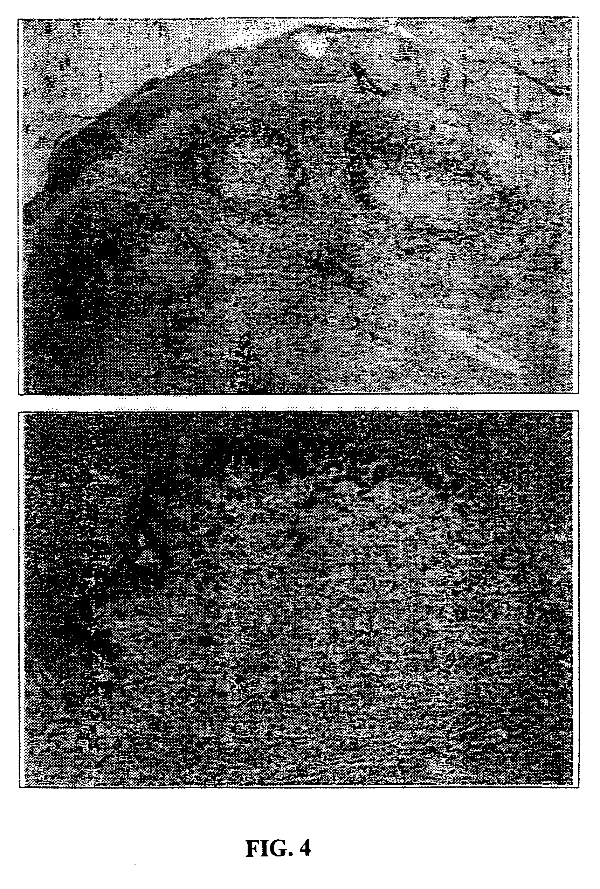

Fig. 4 zeigt das Ergebnis einer histochemischen Untersuchung zur Lokalisation von 8F4-positiven Zellen in der Tonsille.

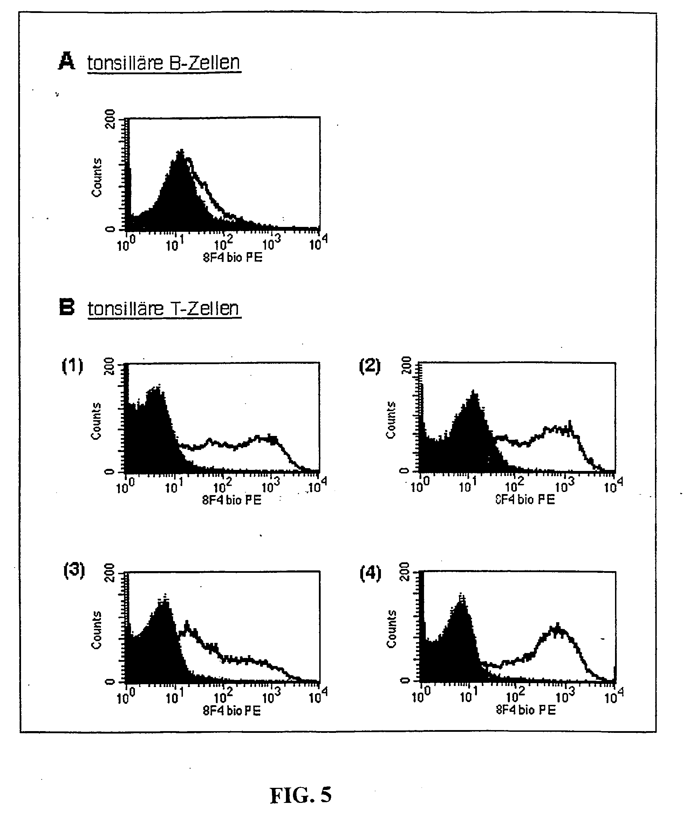

Fig. 5 zeigt das Ergebnis einer Expressions-Analyse von 8F4 auf T- und B-Zellen aus humanen Tonsillen in einer Durchflußzytometrie. "bioPE" bedeutet biotinylierter Antikörper und Streptavidin-Phycoerythrin Sekundärreagenz.

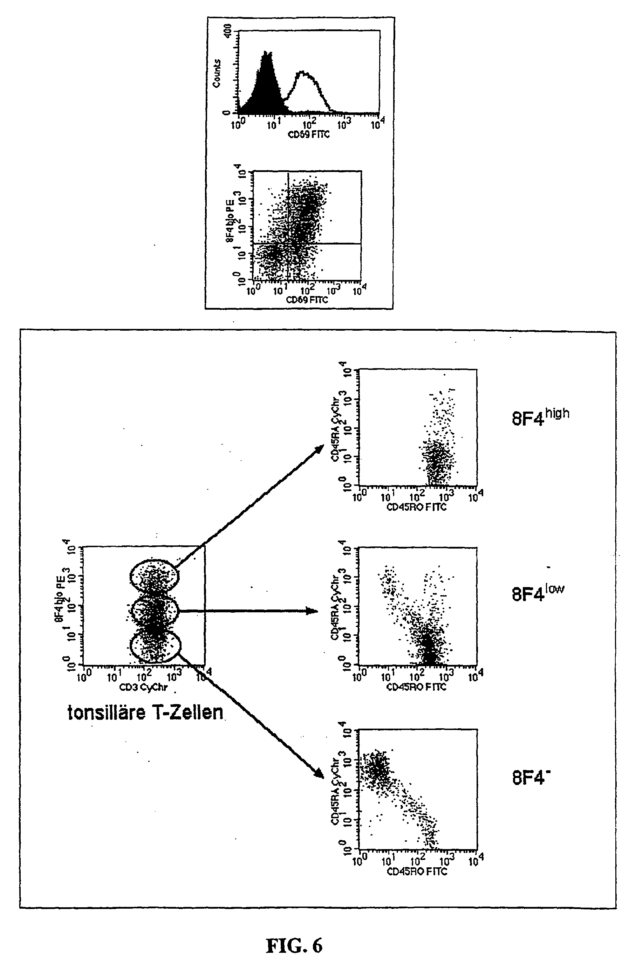

Fig. 6 zeigt die Ko-Expression des 8F4-Moleküls mit anderen Aktivierungsmarkern (CD69, CD45) in einer Durchflußzytometrie.

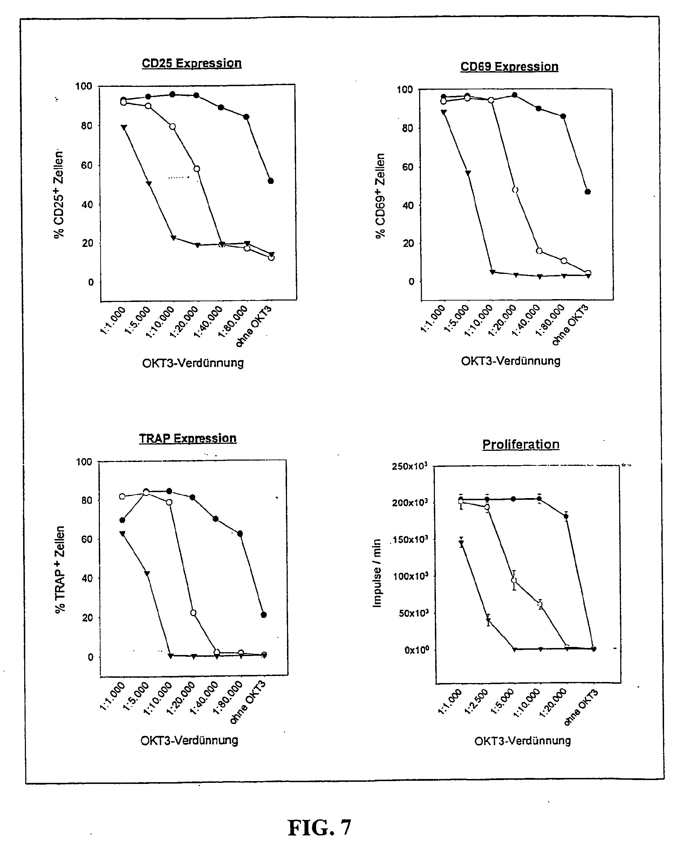

Fig. 7 zeigt schematisch die verstärkte Expression von Aktivierungsmolekülen auf T-Lymphozyten nach Ko-Stimulation durch 8F4. Offene Kreise (O) stehen für 8F4 Antikörper; Dreiecke (◆) stehen für unspezifischer Antikörper gleichen Isotyps; ausgefüllte Kreise (●) stehen für anti-CD28-Antikörper-9.3.

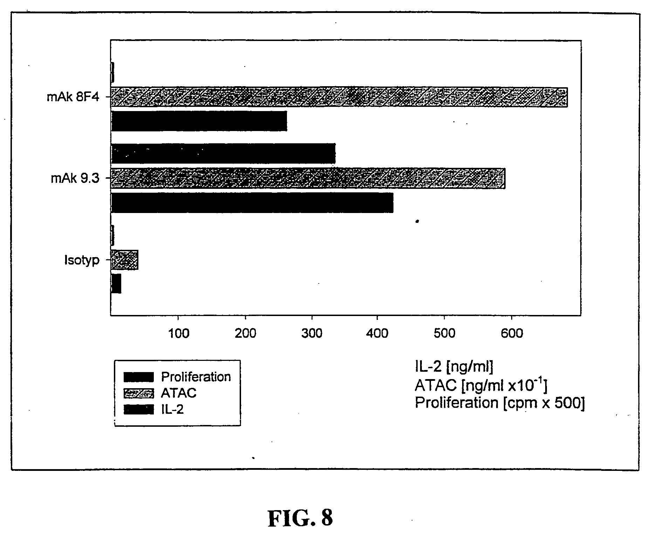

Fig. 8 zeigt einen schematischen Vergleich der ko-stimulierenden Wirkung von 8F4 mit der ko-stimulierenden Wirkung von CD28. "mAk" bedeutet monoklonaler Antikörper; "ATAC" bedeutet "Activation induced T cell derived And Chemokine related"; "cpm" bedeutet radioaktive Zerfälle pro Minute.

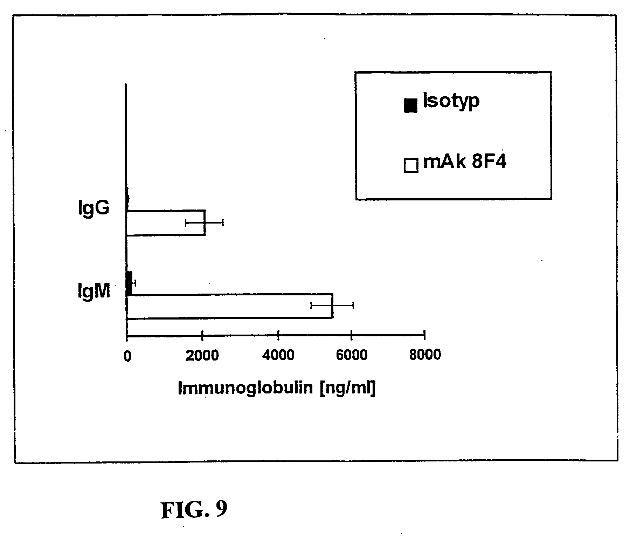

Fig. 9 zeigt schematisch die Verstärkung der Synthese der Antikörper vom IgM und IgG-Typ durch die B-Zellen nach Ko-Stimulation von T-Zellen "ng" bedeutet Nannogramm; "ml" bedeutet Milliliter; "mAk" bedeutet monoklonaler Antikörper.

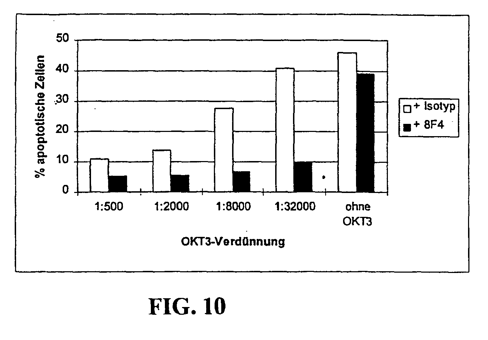

Fig. 10 zeigt schematisch die Verhinderung der aktivierungsinduzierten Apoptose peripherer T-Zellen nach Ko-Stimulation durch 8F4.
Fig. 11 zeigt die Expression des 8F4-Antigens auf der MOLT-4V-Zellinie. MOLT-4V-Zellen wurden mit einem Fluorescein-markierten 8F4-Antikörper (8F4-FITC) gefärbt und in der Durchflußzytometrie untersucht (offene Linie, im Vergleich zu einer Isotypkontrolle (ausgefüllte Linie)).

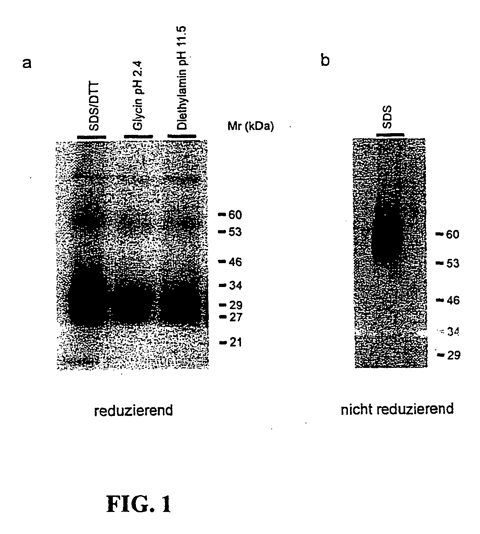

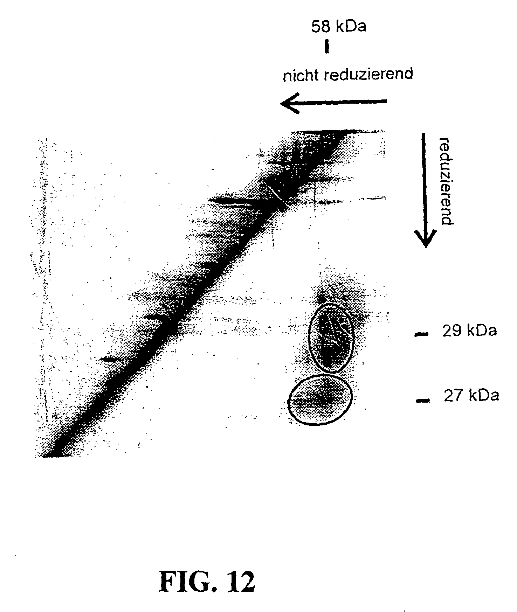

Fig. 12 zeigt die zweidimensionale Gelelektrophorese. Ein MOLT-4V Zell-Lysat aus 300×10⁶ Zellen wurde wie beschrieben immunpräzipitiert. Das Eluat wurde auf einer nicht reduzierenden SDS-PAGE (10% PAA) aufgetrennt und der Bereich um 60 kDa aus dem Gel herausgeschnitten. Zur Reduktion der Disulfidbrücken im 8F4-Molekül wurde das Gelstück für 1 h bei 50°C in 5,3 M Harnstoff, 0,5 M Tris, pH 8,0, 1% SDS, 1% β-Mercaptoethanol inkubiert und die freien Cysteinreste im Molekül mit 10 mM Iodoacetamid (Sigma, Deisenhofen) alkyliert (30 min, 37°C). Das Gelstück wurde für weitere 30 min. in 1× SDS-PAGE Probenpuffer equilibriert und auf einem 12% PAA-SDS-Gel (mit Sammelgel) montiert. Nach elektrophoretischer Auftrennung wurde das Gel einer Silberfärbung unterzogen. Die Lage des 8F4-Proteins wurde durch Oberflächeniodinierung bestimmt (vgl. Fig. 1) und ist durch Umkreisung markiert. (Alle nicht im Detail beschriebenen Prozeduren wurden nach Standardmethoden durchgeführt, siehe z.B. Westermeier, R., Electrophoresis in Practice, VCH Verlagsgesellschaft, Weinheim, 1997).
Fig. 13 zeigt eine Hybridisierung mit Oligo 1 (SEQ ID NO:3). AufNitrocellulose-Filter immobilisierte Lamda-Klone wurden mit Oligo 1 wie in den Beispielen beschrieben hybridisiert. Dargestellt ist die Exposition auf einem Röntgenfilm (Ausschnitt).

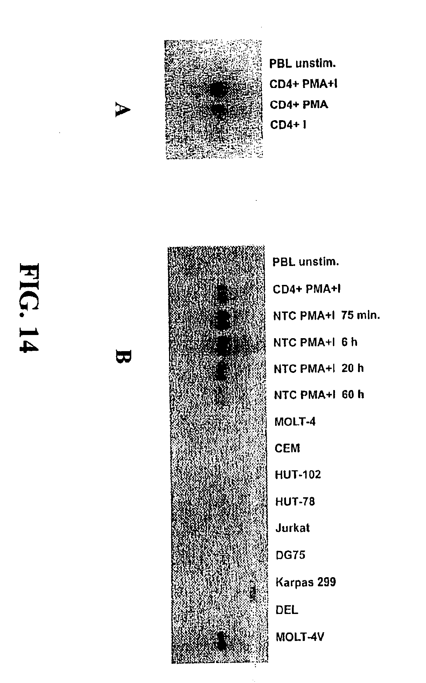

Fig. 14 zeigt eine Northern-Blot Analyse mit der 8F4-cDNA. Die Hybridisierung eines Northern-Blots mit der 8F4-cDNA ergibt eine Bande, die im Gel zwischen der 18S und 28S RNA läuft. In Fig. 14A ist das Verhalten als 2-Signal-abhängiges (s.o.) Aktivierungsantigen gezeigt: Keine Expression in ruhenden lymphoiden Zellen (PBL), starke Expression in PMA+Ionomycin aktivierten CD4+ T-Zellen und deutlich verringerte Expression mit PMA bzw. Ionomycin alleine. Fig. 14B zeigt die Expressionsstärke der mRNA nach unterschiedlichen Stimulationszeiten (T-Zellen (über Nylonwolladhärenz aufgereinigt, NTC), stimuliert mit PMA+Ionomycin). Daneben die MOLT-4-Zellinien (ATCC CRL-1582), die nur eine minimale Expression zeigt, ganz rechts die für die Klonierung verwendete MOLT-4V, die ein deutliches Signal zeigt. Aufgetragen ist außerdem die RNA weiterer Zellinien, auf denen in der durchflußzytometrischen Analyse keine 8F4-Expression nachweisbar war: CEM (ATCC CCL-119), HUT-102 (ATCC TIB-162), HUT-78 (ATCC TIB-161), Jurkat (ATCC TIB-152), DG75 (Deutsche Sammlung von Mikroorganismen und Zellkulturen (DSMZ) ACC83), Karpas 299 (Fischer, P. et al. (1988), Blood, 72:234-240), DEL (Barbey, S. et al. (1990), Int. J. Cancer, 45:546-553)
Fig. 15 zeigt die Aminosäuresequenz des Polypeptids 8F4 (SEQ ID NO:2).
Fig. 16 zeigt die 8F4 cDNA (SEQ ID NO:1).

Die nachstehenden Beispiele erläutern die Erfindung und sind nicht einschränkend zu verstehen.

### Beispiel 1: Generierung des 8F4-Antikörpers

Balb/c Mäuse wurden mit humanen T-Zellen immunisiert, welche vorher für 24 h mit 33 ng/ml des Phorbolesters Phorbolmyristatacetat (PMA), (Sigma, Deisenhofen), und mit 200 ng/ml des Ca²⁺-Ionophors Ionomycin (Sigma, Deisenhofen) aktiviert worden sind (sogenannte "2-Signal-Aktivierung"). Nach dreimaligem Boostern wurden die Milzzellen der Mäuse mit dem Myelom P3X63Ag8.653 (ATCC Nr.CRL-1580) fusioniert und Antikörpersezernierende Hybridome nach Standardmethoden generiert; vgl. Peters und Baumgarten, Monoclonal Antibodies. Springer, Heidelberg, 1992. Das Durchmustern der erhaltenen Antikörper erfolgte auf aktivierten versus ruhenden T-Zellen in der Durchflußzytometrie. Aktivierte ("2-Signal-Aktivierung") und ruhende T-Zellen wurden mit dem Hybridomüberstand inkubiert und anschließend mit einem floureszenzmarkierten Sekundärantikörper markiert; vgl. Shapiro, Practical Flow Cytometry. Wiley-Liss, New York, 1995. Nur die Antikörper, welche Moleküle erkannten, die ausschließlich durch PMA und das Ca²⁺-Ionophors Ionomycin auf der T-Zell-Oberfläche induziert wurden, jedoch nicht durch eines der Agenzien alleine ("2-Signal-Moleküle") wurden für eine weitere Reinigung ausgewählt. Die erhaltenen Antikörper wurden in der Durchflußzytometrie auf Ähnlichkeit bzw. Verschiedenheit zu bekannten Antikörpern gegen Aktivierungsmoleküle (vgl. Tabelle 1) auf T-Zellen untersucht. Kriterien waren hierbei neben der schon oben erwähnten "2 Signal-Abhängigkeit" die Kinetik der Induktion auf stimulierten T-Zellen und die Expression auf verschiedenen Zelllinien.

### Beispiel 2: Immunpräzipitation des 8F4-Antigens

Oberflächenmoleküle von aktivierten humanen T-Zellen wurden mit ¹²⁵I nach Standardmethoden iodiniert und mit dem Antikörper 8F4 nach Standardmethoden immunpräzipitiert; vgl. Goding, Monoclonal Antibodies: Principle and Practice. Academic Press, London, 1996. Für die Immunpräzipitation wurde der Antikörper nach Schneider et al., *Journal of Biological Chemistry* 257 (1982), 10766-10769, an Protein G (Pharmacia, Freiburg) gekoppelt (8F4-Matrix). Das Waschen der Matrix erfolgte nach Schneider et al., siehe vorstehend. Das immunpräzipitierte 8F4-Molekül wurde auf übliche Weise in der SDS-PAGE (nicht reduziert und reduziert) auf seine molekulare Masse analysiert; Goding, siehe vorstehend.

### Beispiel 3: Durchflußzytometrie

Die Analyse der 8F4-tragenden T-Zellen in der Durchflußzytometrie erfolgte nach Standardmethoden; vgl. Shapiro, Practical Flow Cytometry. Wiley-Liss, New York, 1995.

Ausführungsbeispiel 3.1: Durchflußzytometrie nach Induktion des 8F4-Antigens auf CD4⁺-T-Zellen.

CD4⁺-T-Zellen aus dem peripheren Blut wurden mit verschiedenen Agenzien in üblicher Weise stimuliert und auf Expression des 8F4-Moleküls in der Durchflußzytometrie nach üblichem Verfahren untersucht. Die Aktivierungsdauer der T-Zellen betrug mit den verschiedenen Agenzien zwischen 24 Stunden und 144 Stunden. Aktivierungsmodi: Phorbolmyristatacetat (PMA; 33 ng/ml), Ionomycin (200ng/ml), Phytohaemagglutinin (PHA 1,5 mg/ml), OKT3 (monoklonaler Antikörper gegen CD3), gemischte Lymphocytenreaktion (MLR, "mixed lymphocyte reaction" zwichen 50 000 CD4⁺-T-Zellen und 100 000 B-Zellen), mAk 9.3 (monoklonaler Antikörper gegen CD28), Staphylokokken Enterotoxin B (SEB, 0,1 ng/ml). Die Auswertung ergab, daß verschiedene Stimuli geeignet sind, das 8F4-Molekül auf T-Zellen zu induzieren, jedoch in unterschiedlicher Expressionsdichte. Am potentesten sind neben den stark wirksamen pharmakologischen Agenzien PMA und Ionomycin solche Stimuli, die eine ko-stimulatorische Situation darstellen, wie z.B. akzessorische Zellen in der MLR oder der ko-stimulierende mAk 9.3.

Ausführungsbeispiel 3.2: Kinetik der Induktion des 8F4-Antigens auf CD4⁺-T-Zellen nach Aktivierung mit PMA und Ionomycin.

CD4⁺-T-Zellen aus dem peripheren Blut wurden mit PMA (33 ng/ml) und Ionomycin (200 ng/ml) in üblicher Weise stimuliert und nach 0, 4, 8, 12, 24 und 48 Stunden auf die Expression des 8F4-Moleküls in der Durchflußzytometrie in üblicher Weise untersucht. Das Molekül ist bereits nach vier Stunden auf der Oberfläche detektierbar, gehört also zur Klasse der relativ frühen Aktivierungsantigene. Auch nach 48 Stunden wird das Antigen noch gut exprimiert.

Ausführungsbeispiel 3.3: Durchflußzytometrie zur Identifikation von Molekülen, welche an der Induktion von 8F4 in der "mixed lymphocyte reaction" beteiligt sind.

50 000 CD4⁺-T-Zellen aus dem peripheren Blut wurden mit 100 000 allogenen tonsillären B-Zellen für 6 Tage ko-kultiviert (37°C, 5,2% CO₂, 200 µl RPMI 1640 mit 10% FCS in 96-Well-Rundbodenplatten) und danach auf die Expression des 8F4-Moleküls in der Durchflußzytometrie untersucht. Zu Beginn der Kultivierung wurden verschiedene Antikörper (anti-CD80, anti-CD86, anti-MHCII; alle 10 mg/ml) der Kultur hinzugefügt, um die Abhängigkeit der 8F4-Induktion von diesen Molekülen zu überprüfen. Die Expression von 8F4 läßt sich nur durch Blockade der CD86/CD28-Interaktion blockieren, nicht jedoch durch Blockade von CD80. Der Blockade-Effekt ist hierbei noch stärker als die Blockade von MHCII (Positivkontrolle).

Ausführungsbeispiel 3.4: Expression von 8F4 auf T- und B-Zellen aus humanen Tonsillen. B-Zellen bzw. T-Zellen aus tonsillärem Gewebe von verschiedenen Quellen wurden auf übliche Weise gereinigt und in der Durchflußzytometrie auf die Expression des 8F4-Moleküls untersucht. Während auf B-Zellen das Signal nicht eindeutig signifikant war, exprimierten etwa 50-80% der tonsillären T-Zellen das 8F4-Molekül in unterschiedlicher Dichte. Es lassen sich hierbei zwei Populationen unterschiedlich hoher Fluoreszenz (8F4-"high" bzw -"low") erkennen, deren Ausprägung bei den verschiedenen Tonsillen unterschiedlich ist. So weist z.B. Tonsillen eine ausgeprägte 8F4-"low"- und andere Tonsillen eine auspeprägte 8F4-"high"-Population auf.

Ausführungsbeispiel 3.5: Ko-Expression des 8F4-Moleküls mit anderen Aktivierungsmarkern.
Aus humanen Tonsillen gereinigte T-Zellen wurden in der 2-Farben-Durchflußzytometrie auf die Ko-Expression des 8F4-Moleküls mit anderen Aktivierungsmarkern analysiert. In Tonsillen wird 8F4 ko-exprimiert mit CD69 wie auch mit Varianten des CD45-Moleküls. Hierbei sind die 8F4-"high"-Zellen eindeutig mit einer CD45RO-Expression korreliert, während die 8F4-negativen Zellen den Phänotyp CD45RA tragen. CD45RA wird hauptsächlich von sogenannten "naiven" T-Zellen exprimiert, wohingegen CD45RO mit einer Effektorzellfunktion assoziiert ist. Es handelt sich also bei den 8F4⁺-Zellen hauptsächlich um "reife" T-Zellen. CD45RO und CD45RA sind Isoformen von CD45.

### Beispiel 4: Lokalisation von 8F4-positiven Zellen in der Tonsille.

Tonsilläres Gewebe in Gefrierschnitten wurde mit dem 8F4-Antikörper in der APAAP-Technik (alkalische-Phosphatase-anti-alkalische-Phosphatase) nach Standardverfahren angefärbt. 8F4⁺-Zellen wurden vorzugsweise im Keimzentrum der Tonsillen vorgefunden, aber z. T. auch in der T-Zell-Zone der Tonsillen.

### Beispiel 5: Ko-Stimulation von T-Lymphozyten

96-Well-Platten wurden mit einem Ziegen-anti-Maus-Ig-Antikörper beschichtet (20 µg/ml), gewaschen, und mit dem anti-CD3 monoklonalen Antikörper OKT3 (verschiedene Verdünnungen eines Aszites) und dem erfindungsgemäßen 8F4-Antikörper (2 µg/ml) beladen. Als Isotypkontrolle wurden der OKM1 Antikörper oder der 2A11 Antikörper (beide 2 µg/ml) verwendet.

Ausführungsbeispiel 5.1: Verstärkte Expression von Aktivierungsmolekülen auf T-Lymphozyten nach Ko-Stimulation durch 8F4.

Gereinigte CD4⁺-T-Zellen aus dem peripheren Blut wurden mit verschiedenen Konzentrationen des monoklonalen Antikörpers OKT3 aktiviert und gleichzeitig mit dem 8F4-Antikörper oder einem unspezifischen Antikörper gleichen Isotyps ko-stimuliert. Als Vergleich wurde die Kostimulation mit dem anti-CD28-Antikörper-9.3, einem der stärksten bekannten ko-stimulatorischen Antikörper, durchgeführt. Selbst bei optimaler Stimulation über CD3 ist sowohl mit dem mAk 8F4 als auch mit mAk 9.3 noch ein Ko-stimulatorischer Effekt zu sehen. Im suboptimalen OKT3-Bereich, d.h. dem Bereich, in dem ohne Kostimulation keine volle T-Zellaktivierung mehr erzielt werden kann, können beide Antikörper die Expression anderer Aktivierungsantigene um den Faktor 4 bis 100 steigern, wobei die Wirkung des anti-CD28-Antikörpers auch noch bei sehr hohen OKT3-Verdünnungen sichtbar wird. Dies ist darauf zurückzuführen, daß bei sehr schwacher OKT3-Stimulation das 8F4-Antigen nicht mehr auf die Zelloberfläche gebracht und somit auch nicht vom mAk 8F4 kreuzvernetzt werden kann.

Ausführungsbeispiel 5.2: Vergleich der ko-stimulierenden Wirkung von 8F4 mit der ko-stimulierenden Wirkung von CD28.

Gereinigte CD8⁺-T-Zellen wurden für 51 h mit einer suboptimalen Konzentration des monoklonalen Antikörpers OKT3 stimuliert. Als Ko-Stimulatoren wurden Antikörper 8F4, Antikörper 9.3 (anti-CD28) und Isotypkontrollen eingesetzt (jeweils 2 µg/ml). Nach Ablauf der Stimulationsdauer wurde die Proliferationsrate der T-Zellen durch ³H-Thymidin-Einbau bestimmt. In Parallelkulturen wurde der Überstand entfernt und die Konzentration der Zytokine ATAC/Lymphotactin und IL-2 bestimmt. Bezüglich der IL-2-Synthese unterscheiden sich 8F4 und CD28 sehr stark voneinander. CD28-Ko-Stimulation führt, wie auch im Stand der Technik beschrieben (Chambers und Allison, *Current Opinion in Immunology* 9 (1997), 396-404), zu einer sehr starken IL-2 Sekretion. Mit 8F4 hingegen liegt die IL-2 Produktion unterhalb der Nachweisgrenze. Die Proliferation ist jedoch in beiden Ansätzen vergleichbar, das autokrine Wachstum der T-Zellen muß also bei 8F4-Ko-Stimulation auf andere Faktoren zurückgeführt werden. Auch in bezug auf die Sekretion des Lymphokins ATAC unterscheiden sich die beiden Antikörper in der ko-stimulatorischen Wirkung kaum.

### Beispiel 6: Bestimmung der von B-Zellen synthetisierten Immunglobuline nach Interaktion mit 8F4-ko-stimulierten T-Zellen

96-Well-Platten wurden mit einem Ziegen-anti-Maus-Ig-Antikörper beschichtet (20 µg/ml), gewaschen, und mit dem anti-CD3 monoklonalen Antikörper OKT 3 (1:500 bis 1:80 000 Aszites) und dem erfindungsgemäßen 8F4-Antikörper (2 µg/ml) beladen. Als Isotypkontrolle wurde der OKM1-Antikörper oder der 2A11-Antikörper verwendet. In einigen Experimenten wurde zum Vergleich eine Ko-Stimulation mit einem CD28-spezifischen Antikörper ("9.3") durchgeführt; vgl. Hara et al., *Journal of Experimental Medicine* 161 (1985), 1513-1524. In die so vorbehandelten Kulturplatten wurden pro well 50 000 gereinigte (Magnetobeads, Dynal, Hamburg) CD4⁺-T-Zellen (>95% Reinheit) aus dem peripheren Blut und 25 000 allogene tonsilläre B-Zellen (negativ-Selektion durch T-Zell-Rosettierung mit Schafserythrocyten, 96% Reinheit) pipettiert und für 8 Tage ko-kultiviert. Nach dieser Zeitdauer wurde der Überstand entnommen und auf die Konzentration sezernierter Immunglobuline vom IgM- und IgG-Typ im ELISA auf übliche Weise analysiert; vgl. Nishioka and Lipsky, *Journal of Immunology* 153 (1994), 1027-1036.

Ausführungsbeispiel 6.1: Verstärkung der Synthese der Antikörper vom IgM- und IgG-Typ durch die B-Zellen nach Ko-Stimulation von T-Zellen.

Gereinigte CD4⁺-T-Zellen aus dem peripheren Blut wurden für 8 Tage mit allogenen B-Zellen aus Tonsillen in üblicher Weise ko-kultiviert. Bei einer suboptimalen Stimulation der T-Zellen mit dem OKT3-Antikörper verstärkt die Ko-Stimulation der T-Zellen durch 8F4 die Sekretion der IgM- und IgG-Immunglobuline um den Faktor 40.

### Beispiel 7: Verhinderung der aktivierungsinduzierten Apoptose peripherer T-Zellen nach Ko-Stimulation durch 8F4.

Periphere T-Zellen (über Nylonwolladhärenz in üblicher Weise aufgereinigt) wurden für 20 h mit PHA (1.5 mg/ml) stimuliert und über 6 Tage mit IL-2 kultiviert. Anschließend wurden die Zellen durch OKT3 mit und ohne Ko-Stimulation durch mAk 8F4 (2 µg/ml) restimuliert. Die Apoptose wurde durch Anfärbung der DNA mit Propidiumiodid in der Durchflußzytometrie (FACS) bestimmt. Bei suboptimaler Stimulation über den T-Zellrezeptor-Komplex kann Ko-Stimulation über 8F4 den Anteil apoptotischer Zellen um den Faktor 4 senken.

### Beispiel 8: Klonierung der für das 8F4-Protein kodierenden cDNA

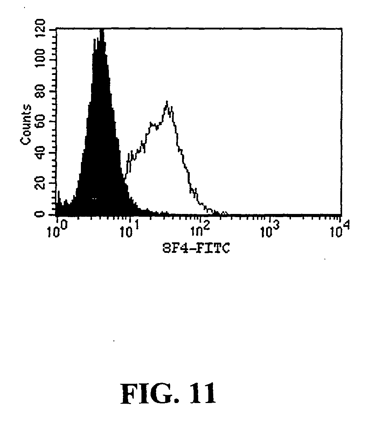

Durch Färbung mit einem Fluoreszenzfarbstoff-gekoppelten 8F4 Antikörper wurde in der Durchflußzytometrie eine Zellinie (MOLT-4V) identifiziert, die das 8F4-Antigen konstitutiv exprimiert (Fig. 11). Bei der Linie MOLT-4V handelt es sich um eine Variante der humanen T-Zellinie MOLT-4 (American Type Culture Collection (ATCC) CRL-1582).

Diese Zellinie wurde für die präparative Aufreinigung des 8F4-Antigens mit Hilfe des monoklonalen Antikörpers verwendet:
Die Zellen wurden im großen Maßstab (150 1) in Rollerkulturflaschen kultiviert, abzentrifugiert und die zellulären Proteine mit einem Lysepuffer (50 mM Tris, pH 8,0, 150 mM NaCl, 1 mM EDTA, 1 mM PMSF (Sigma, Deisenhofen), 1% NP-40 (Boehringer, Mannheim)) extrahiert. Zellkerne und andere unlösliche Bestandteile wurden durch Ultrazentrifugation entfernt. Das so gewonnene Zell-Lysat wurde für 2 h mit Sepharose CL4-B (Pharmacia, Freiburg) präinkubiert, um unspezifisch an Sepharose bindende Proteine zu entfernen. Anschließend erfolgte die Inkubation mit der bereits in Beispiel 2 beschriebenen 8F4-Immunaffinitätsmatrix (4 h bei 4°C). Die Matrix wurde in eine Säule gefüllt und nun mehrfach unter Bedingungen gewaschen, die eine ausschließliche Entfernung von unspezifisch bindenden Proteinen bedingt (l. 50 mM Tris, pH 8,0, 300 mM NaCl, 1 mM EDTA, 1 mM PMSF, 0,5% NP-40; 2. 50 mM Tris, pH 8,0, 150 mM NaCl, 1 mM EDTA, 1 mM PMSF, 0,5% NP-40, 0,1% SDS; 3. 0,2 M Glycin pH 4,0, 0,5% CHAPS (Merck, Darmstadt)). Die Elution des 8F4-Antigens von der Matrix erfolgte mit 0,2 M Glycin, pH 2,5, 0,5% CHAPS. Das Eluat wurde durch Ultrafiltration eingeengt (Amicon Centricon 10, Millipore, Eschborn).

Um eine weitere Aufreinigung des 8F4 Moleküls zu erzielen, wurde die dimere Struktur des Moleküls (siehe Fig. 1) in einer zweidimensionalen Gelelektrophorese (nicht reduzierend/reduzierend) ausgenutzt: Da die meisten Proteine als Monomer vorkommen, laufen sie in der Gelelektrophorese auf einer Diagonale, das 8F4-Molekül dagegen läuft in der 1. Dimension (nicht reduzierend) bei 55-60 kDa und in der 2. Dimension (reduzierend) bei 27 und 29 kDa (Fig. 12).

Für die präparative Auftrennung wurden die Immunpräzipitate von jeweils 20×10⁹ Zellen wie vorstehend bei der Beschreibung der Fig. 12 dargestellt in der Zweidimensionalen Gelelektrophorese aufgetrennt, das Gel mit Coomassie Blau G250 (Biorad, München) gefärbt und die in Fig. 12 bezeichneten Areale aus dem Gel getrennt ausgeschnitten (8F4-27kDa bzw. 8F4-29 kDa).

Für die Peptidmikrosequenzierung wurden die Proteine aus jeweils 4 Gelstücken mit Trypsin verdaut und aus dem Gel eluiert. Die tryptischen Fragmente wurden über HPLC aufgetrennt und einzelne Fraktionen einer Edman-Degradation unterzogen (Verfahren ausführlich beschrieben in Groettrup, M. et al. (1996), Eur. J. Immunol., 26:863-869).
Aus der Sequenzierung der 8F4-29kDa Probe wurde neben Bruchstücken bekannter Proteine eine Peptidsequenz XRLTDVT gefunden, für die in sämtlichen Proteindatenbanken kein Korrelat im humanen Bereich gefunden wurde.

Eine eindeutige Rückübersetzung einer Proteinsequenz in eine DNA-Sequenz ist nicht möglich. So ergibt die Rückübersetzung der obigen Peptidsequenz in ein Oligonucleotid mit 17 Nucleotiden eine Zahl von 2048 Permutationen. Ein spezielles Verfahren (Wozney, J.M. (1990), Methods Enzymol. 182:738-751) ermöglicht jedoch das Screening einer cDNA Bank mit degenierten Oligonucleotiden. Auf Basis der gefundenen Peptidsequenz wurden 2 Oligonucleotide (Oligo 1 (SEQ ID NO:3): MGN CTS ACN GAY GTN AC, 512 Permutationen; Oligo 2 (SEQ ID NO:4): MGN YTD ACN GAY GTN AC, 1024 Permutationen) synthetisiert.

Für das Screening wurde eine cDNA Bank aus der auch für die Proteinaufreinigung verwendeten MOLT-4V Zellinie konstruiert:
Gesamt-RNA wurde nach der Guanidinium/CsCl-Methode (Chirgwin, J.M. et al. (1979), Biochemistry 18:5294-5299) isoliert, mRNA über Oligo-dT-Cellulose-Säulen (Gibco BRL, Eggenstein) angereichert. Die Erst- und Zweit-Strang cDNA Synthese wurde unter Verwendung eines kommerziellen cDNA-Synthesesystems (Gibco BRL, Eggenstein) unter Verwendung von Oligo-dT-Primern nach Angaben des Herstellers durchgeführt. Die cDNA wurde wurde über EcoRI-Adaptoren in den Lambda ZAPII Vektor (Stratagene, Heidelberg) ligiert.

Die cDNA-Bank wurde gemäß Standardmethoden (Vogeli, G. and Kaytes, P.S. (1987), Methods Enzymol., 152:407-515) plattiert und die Lamda-DNA auf Nitrocellulose-Filtern (Optitran BA-S 85, Schleicher & Schuell, Dassel) immobilisiert.
Die oben genannten Oligonucleotide wurden unter Verwendung von T4 Polynucleotidkinase (NEBL, Schwalbach) und γ-³²P ATP (NEN Du Pont, Brüssel) radioaktiv markiert (Wallace, R.B. and Miyada, C.G. (1987), Methods Enzymol., 152:432-442).

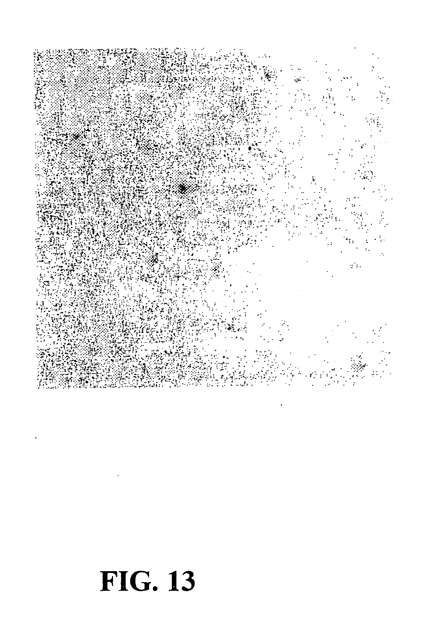

Die Hybridisierung der Filter erfolgte in einem für degenerierte Oligonucleotide beschriebenen Puffer (Wozney, J.M. (1990), Methods Enzymol. 182:738-751) mit 3 M Tetramethylammoniumchlorid (Roth, Karlsruhe) bei 48°C. Die Filter wurden, wie in der o.g. Referenz beschrieben, gewaschen, wobei die Waschtemperatur 50°C betrug. Nach Exposition der Filter auf einem Röntgenfilm zeigten sich ca. 50 positive Klone pro 100 000 plattierter Phagen (Fig. 13).

6 Klone wurden weiter charakterisiert, indem sie nach der vom Herstellers des Vektors (Stratagene, Heidelberg) beschriebenen Methode durch in vivo Excision in einen Plasmidvektor überführt wurden und mit T3 und T7 Primern ansequenziert wurden (BigDye Terminator Cycle Sequencing Kit, Applied Biosystems, Foster City, USA). Einer der Klone enthielt eine Sequenz, deren Translation genau die gesuchte Peptidsequenz ergab. Dieser Klon wurde zur Hybridisierung eines Northern Blots (Fig. 14) verwendet (Kroczek, R.A. (1993), J. Chromatogr., 618, 133-145). Das Expressionsmuster der mRNA entsprach genau der Expression des 8F4-Moleküls, wie es aus durchflußzytometrischen Untersuchungen mit dem monoklonalen Antikörper bekannt war. Da der gefundene Klon nur das 3'-Ende der gesuchten cDNA enthielt, wurde ein 5'-gelegenes Fragment zur Isolierung der gesamten 8F4 cDNA verwendet. Mehrere Klone wurden auf beiden Strängen sequenziert.

Die 8F4 cDNA (2641 Nucleotide) ist in Fig. 16 und im Sequenzprotokoll unter SEQ ID NO:1 dargestellt und codiert für ein Protein mit 199 Aminosäuren (Nucleotide 68-664), dargestellt in Fig. 15 und im Sequenzprotokoll unter SEQ ID NO:2. Die Sequenzierung mehrerer unabhängiger Klone aus der cDNA-Bank ergab einige Abweichungen von der hier gezeigten Sequenz, die jedoch alle in der 3'-untranslatierten Region liegen:
Pos. 909-910: Deletion
Pos. 1631: T->C
Pos. 2074: G->T
Pos. 2440: G->C
Pos. 2633: alternative Polyadenylierungsstelle

**Tab.1:**

| Tabelle 1 gibt eine Übersicht über die verwendeten Antikörper (Klon), deren Herkunftsquelle (Quelle), die Spezifität gegen ihr jeweiliges Antigen (Spezifität) und gegebenenfalls ihre Markierung (Label) wider. | | | | |
|---|---|---|---|---|
| **Spezifität** | **Label** | **Isotyp** | **Klon** | **Quelle** |
| **CD3** | Cy-Chrome | IgG1 | UCHT1 | Pharmingen, Hamburg |
| **CD3** | - | IgG2a | OKT3 | ATCC CRL-8001 |
| **CD11b** | - | IgG2b | OKM1 | ATCC CRL-8026 |
| **CD25** | FITC | IgG2a | B1.49.9 | Immunotech, Hamburg |
| **CD28** | - | IgG2a | 9.3 | Immunex Corp., Seattle |
| **CD45RA** | Cy-Chrome | IgG2b | HI100 | Pharmingen, Hamburg |
| **CD45RO** | FITC | IgG2a | UCHLI | Immunotech, Hamburg |
| **CD69** | FITC | IgG1 | FN50 | Pharmingen, Hamburg |
| **CD80** | - | IgG1 | L307.4 | Becton Dickinson, Heidelberg |
| **CD86** | - | IgG2b | IT2.2 | Pharmingen, Hamburg |
| **CD154** | FITC | IgG1 | TRAP-1 | Hybridom¹ |
| **MHCII** | - | IgG2a | L243 | ATCC HB-55 |
| **8F4** | - | IgG1 | 8F4 | Hybridom¹ |
| **8F4** | Biotin | IgG1 | 8F4 | Hybridom¹ |
| **Isotyp IgG1** | - | IgG1 | 2A11 | Hybridom^{1,2} |
| **Isotyp IgG1** | FITC | IgG1 | 2A11 | Hybridom^{1,2} |
| **Isotyp IgG1** | Biotin | IgG1 | ASA-1 | Hybridom¹ |

| | | | | |
|---|---|---|---|---|
| ¹ Die Hybridomzellinie wurde auf übliche Weise generiert, der Antikörper aufgereinigt und gegebenenfalls markiert. ² gerichtet gegen ein synthetisches Peptid | | | | |

Die in den Beispielen verwendeten Antiseren und Sekundärreagenzien wurden bezogen von: Ziege-anti-Maus-Ig, FITC-konjugiert, von Jackson Immuno Research Lab., USA; Streptavidin, PE-konjugiert, von Jackson Immuno Research Lab., USA; Kaninchen-anti-Maus-Ig-Fraktion, von Sigma, Deisenhofen.

### SEQUENZPROTOKOLL

(1) ALLGEMEINE ANGABEN:
   (i) ANMELDER:
      (A) NAME: Bundesrepublik Deutschland, letztvertreten durch den Direktor des Robert-Koch-Instituts
      (B) STRASSE: Nordufer 20
      (C) ORT: Berlin
      (D) BUNDESLAND: Berlin
      (E) LAND: Deutschland
      (F) POSTLEITZAHL: 13353
   (ii) BEZEICHNUNG DER ERFINDUNG: Ko-stimulierendes Polypeptid von T-Zellen, monoklonale Antikörper sowie die Herstellung und deren Verwendung
   (iii) ANZAHL DER SEQUENZEN: 4
   (iv) COMPUTER-LESBARE FASSUNG:
      (A) DATENTRAEGER: Floppy disk
      (B) COMPUTER: IBM PC compatible
      (C) BETRIEBSSYSTEM: PC-DOS/MS-DOS
      (D) SOFTWARE: PatentIn Release #1.0, Version #1.30 (EPA)
   (v) DATEN DER JETZIGER ANMELDUNG: ANMELDENUMMER:
(2) ANGABEN ZU SEQ ID NO: 1:
   (i) SEQUENZKENNZEICHEN:
      (A) LÄNGE: 2641 Basenpaare
      (B) ART: Nucleotide
      (C) STRANGFORM: Doppelstrang
      (D) TOPOLOGIE: linear
   (ii) ART DES MOLEKUELS: cDNA
   (xi) SEQUENZBESCHREIBUNG: SEQ ID NO: 1:
   CGAGAGCCTG AATTCACTGT CAGCTTTGAA CACTGAACGC GAGGACTGTT AACTGTTTCT     60
   GGCAAACATG AAGTCAGGCC TCTGGTATTT CTTTCTCTTC TGCTTGCGCA TTAAAGTTTT    120
   AACAGGAGAA ATCAATGGTT CTGCCAATTA TGAGATGTTT ATATTTCACA ACGGAGGTGT    180
   ACAAATTTTA TGCAAATATC CTGACATTGT CCAGCAATTT AAAATGCAGT TGCTGAAAGG    240
   GGGGCAAATA CTCTGCGATC TCACTAAGAC AAAAGGAAGT GGAAACACAG TGTCCATTAA    300
   GAGTCTGAAA TTCTGCCATT CTCAGTTATC CAACAACAGT GTCTCTTTTT TTCTATACAA    360
   CTTGGACCAT TCTCATGCCA ACTATTACTT CTGCAACCTA TCAATTTTTG ATCCTCCTCC    420
   TTTTAAAGTA ACTCTTACAG GAGGATATTT GCATATTTAT GAATCACAAC TTTGTTGCCA    480
   GCTGAAGTTC TGGTTACCCA TAGGATGTGC AGCCTTTGTT GTAGTCTGCA TTTTGGGATG    540
   CATACTTATT TGTTGGCTTA CAAAAAAGAA GTATTCATCC AGTGTGCACG ACCCTAACGG    600
   TGAATACATG TTCATGAGAG CAGTGAACAC AGCCAAAAAA TCTAGACTCA CAGATGTGAC 660
   CCTATAATAT GGAACTCTGG CACCCAGGCA TGAAGCACGT TGGCCAGTTT TCCTCAACTT 720
   GAAGTGCAAG ATTCTCTTAT TTCCGGGACC ACGGAGAGTC TGACTTAACT ACATACATCT 780
   TCTGCTGGTG TTTTGTTCAA TCTGGAAGAA TGACTGTATC AGTCAATGGG GATTTTAACA 840
   GACTGCCTTG GTACTGCCGA GTCCTCTCAA AACAAACACC CTCTTGCAAC CAGCTTTGGA 900
   GAAAGCCCAG CTCCTGTGTG CTCACTGGGA GTGGAATCCC TGTCTCCACA TCTGCTCCTA 960
   GCAGTGCATC AGCCAGTAAA ACAAACACAT TTACAAGAAA AATGTTTTAA AGATGCCAGG 1020
   GGTACTGAAT CTGCAAAGCA AATGAGCAGC CAAGGACCAG CATCTGTCCG CATTTCACTA 1080
   TCATACTACC TCTTCTTTCT GTAGGGATGA GAATTCCTCT TTTAATCAGT CAAGGGAGAT 1140
   GCTTCAAAGC TGGAGCTATT TTATTTCTGA GATGTTGATG TGAACTGTAC ATTAGTACAT 1200
   ACTCAGTACT CTCCTTCAAT TGCTGAACCC CAGTTGACCA TTTTACCAAG ACTTTAGATG 1260
   CTTTCTTGTG CCCTCAATTT TCTTTTTAAA AATACTTCTA CATGACTGCT TGACAGCCCA 1320
   ACAGCCACTC TCAATAGAGA GCTATGTCTT ACATTCTTTC CTCTGCTGCT CAATAGTTTT 1380
   ATATATCTAT GCATACATAT ATACACACAT ATGTATATAA AATTCATAAT GAATATATTT 1440
   GCCTATATTC TCCCTACAAG AATATTTTTG CTCCAGAAAG ACATGTTCTT TTCTCAAATT 1500
   CAGTTAAAAT GGTTTACTTT GTTCAAGTTA GTGGTAGGAA ACATTGCCCG GAATTGAAAG 1560
   CAAATTTATT TTATTATCCT ATTTTCTACC ATTATCTATG TTTTCATGGT GCTATTAATT 1620
   ACAAGTTTAG TTCTTTTTGT AGATCATATT AAAATTGCAA ACAAAATCAT CTTTAATGGG 1680
   CCAGCATTCT CATGGGGTAG AGCAGAATAT TCATTTAGCC TGAAAGCTGC AGTTACTATA 1740
   GGTTGCTGTC AGACTATACC CATGGTGCCT CTGGGCTTGA CAGGTCAAAA TGGTCCCCAT 1800
   CAGCCTGGAG CAGCCCTCCA GACCTGGGTG GAATTCCAGG GTTGAGAGAC TCCCCTGAGC 1860
   CAGAGGCCAC TAGGTATTCT TGCTCCCAGA GGCTGAAGTC ACCCTGGGAA TCACAGTGGT 1920
   CTACCTGCAT TCATAATTCC AGGATCTGTG AAGAGCACAT ATGTGTCAGG GCACAATTCC 1980
   CTCTCATAAA AACCACACAG CCTGGAAATT GGCCCTGGCC CTTCAAGATA GCCTTCTTTA 2040
   GAATATGATT TGGCTAGAAA GATTCTTAAA TATGTGGAAT ATGATTATTC TTAGCTGGAA 2100
   TATTTTCTCT ACTTCCTGTC TGCATGCCCA AGGCTTCTGA AGCAGCCAAT GTCGATGCAA 2160
   CAACATTTGT AACTTTAGGT AAACTGGGAT TATGTTGTAG TTTAACATTT TGTAACTGTG 2220
   TGCTTATAGT TTACAAGTGA GACCCGATAT GTCATTATGC ATACTTATAT TATCTTAAGC 2280
   ATGTGTAATG CTGGATGTGT ACAGTACAGT ACTGAACTTG TAATTTGAAT CTAGTATGGT 2340
   GTTCTGTTTT CAGCTGACTT GGACAACCTG ACTGGCTTTG CACAGGTGTT CCCTGAGTTG 2400
   TTTGCAGGTT TCTGTGTGTG GGGTGGGGTA TGGGGAGGAG AACCTTCATG GTGGCCCACC 2460
   TGGCCTGGTT GTCCAAGCTG TGCCTCGACA CATCCTCATC CCCAGCATGG GACACCTCAA 2520
   GATGAATAAT AATTCACAAA ATTTCTGTGA AATCAAATCC AGTTTTAAGA GGAGCCACTT    2580
   ATCAAAGAGA TTTTAACAGT AGTAAGAAGG CAAAGAATAA ACATTTGATA TTCAGCAACT    2640
   G                                                                    2641
(2) ANGABEN ZU SEQ ID NO: 2:
   (i) SEQUENZKENNZEICHEN:
      (A) LÄNGE: 199 Aminosäuren
      (B) ART: Aminosäure
      (D) TOPOLOGIE: linear
   (ii) ART DES MOLEKUELS: Protein
   (xi) SEQUENZBESCHREIBUNG: SEQ ID NO: 2:
   Met Lys Ser Gly Leu Trp Tyr Phe Phe Leu Phe Cys Leu Arg Ile Lys
   1               5                   10                  15
   Val Leu Thr Gly Glu Ile Asn Gly Ser Ala Asn Tyr Glu Met Phe Ile
               20                  25                  30
   Phe His Asn Gly Gly Val Gln Ile Leu Cys Lys Tyr Pro Asp Ile Val
           35                  40                  45
   Gln Gln Phe Lys Met Gln Leu Leu Lys Gly Gly Gln Ile Leu Cys Asp
       50                  55                  60
   Leu Thr Lys Thr Lys Gly Ser Gly Asn Thr Val Ser Ile Lys Ser Leu
   65                  70                  75                  80
   Lys Phe Cys His Ser Gln Leu Ser Asn Asn Ser Val Ser Phe Phe Leu
                   85                  90                  95
   Tyr Asn Leu Asp His Ser His Ala Asn Tyr Tyr Phe Cys Asn Leu Ser
               100                 105                 110
   Ile Phe Asp Pro Pro Pro Phe Lys Val Thr Leu Thr Gly Gly Tyr Leu
           115                 120                 125
   His Ile Tyr Glu Ser Gln Leu Cys Cys Gln Leu Lys Phe Trp Leu Pro
       130                 135                 140
   Ile Gly Cys Ala Ala Phe Val Val Val Cys Ile Leu Gly Cys Ile Leu
   145                 150                 155                 160
   Ile Cys Trp Leu Thr Lys Lys Lys Tyr Ser Ser Ser Val His Asp Pro
                   165                 170                 175
   Asn Gly Glu Tyr Met Phe Met Arg Ala Val Asn Thr Ala Lys Lys Ser
               180                 185                 190
   Arg Leu Thr Asp Val Thr Leu
           195
(2) ANGABEN ZU SEQ ID NO: 3:
   (i) SEQUENZKENNZEICHEN:
      (A) LÄNGE: 17 Basenpaare
      (B) ART: Nucleotide
      (C) STRANGFORM: Einzelstrang
      (D) TOPOLOGIE: linear
   (ii) ART DES MOLEKUELS: DNA
   (iii) HYPOTHETISCH: Ja
   (iv) ANTISENSE: NEIN
   (xi) SEQUENZBESCHREIBUNG: SEQ ID NO:3:
      MGNCTSACNG AYGTNAC 17
(2) ANGABEN ZU SEQ ID NO: 4:
   (i) SEQUENZKENNZEICHEN:
      (A) LÄNGE: 17 Basenpaare
      (B) ART: Nucleotide
      (C) STRANGFORM: Einzelstrang
      (D) TOPOLOGIE: linear
   (ii) ART DES MOLEKUELS: DNA
   (iii) HYPOTHETISCH: Ja
   (iv) ANTISENSE: NEIN
   (xi) SEQUENZBESCHREIBUNG: SEQ ID NO: 4:
      MGNYTDACNG AYGTNAC 17

## Patentansprüche

1. Ein Maus-Antikörper, der ein humanes ko-stimulierendes Molekül
a) mit der biologischen Aktivität der Ko-Stimulation von T-Zellen,
b) das auf aktivierten CD4⁺- und CD8⁺-T-Lymphozyten, aber nicht auf ruhenden oder aktivierten B-Zellen, Granulozyten, Monozyten, NK-Zellen oder dendritischen Zellen vorkommt,
c) das zwei Polypeptidketten aufweist, wobei das genannte Molekül ein Molekulargewicht von etwa 55 bis 60 kDa hat, bestimmt in einer nicht reduzierenden SDS-Polyacrylamidgelelektrophorese, und wobei die zwei Polypeptidketten des genannten Moleküls ein Molekulargewicht von etwa 27 kDa und etwa 29 kDa haben, gemessen in einer reduzierenden SDS-Polyacrylamidgelelektrophorese, und
d) enthaltend eine Aminosäuresequenz, die mindestens 80% Homologie mit der 199 Aminosäuren umfassenden Sequenz in Fig. 15 (SEQ ID NO:2) aufweist oder ein biologisch aktives Fragment davon, das ebenfalls eine ko-stimulierende Wirkung auf T-Zell-Lymphozyten zeigt, oder enthaltend die Aminosäuresequenz gemäß Fig. 15 (SEQ ID NO:2) oder ein biologisch aktives Fragment davon, das ebenfalls eine ko-stimulierende Wirkung auf T-Zell-Lymphozyten zeigt,
spezifisch erkennt, wobei der Antikörper 2-Signal-aktivierte, jedoch nicht ruhende T-Lymphozyten erkennt und wobei der Antikörper die biologische Aktivität des ko-stimulierenden Moleküls hemmt oder zusammen mit dem monoklonalen anti-CD3-Antikörper OKT3 menschliche T-Lymphozyten ko-stimuliert.

2. Ein Antikörper gemäß Anspruch 1, der ein monoklonaler Antikörper ist.

3. Ein Antikörper gemäß Anspruch 2, wobei die T-Lymphozyten CD4⁺-T-Zellen sind.

4. Ein Antikörper gemäß Anspruch 2, wobei die T-Lymphozyten CD8⁺-T-Zellen sind.

5. Ein Antikörper gemäß einem der Ansprüche 1 bis 4, wobei der Antikörper ein Antigen von ungefähr 55-60 kDa in einer nicht-reduzierenden SDS-Polyacrylamidgelelektrophorese auf aktivierten T-Lymphozyten erkennt.

6. Ein Antikörper gemäß einem der Ansprüche 1 bis 4, wobei der Antikörper ein Antigen von ungefähr 27 und ungefähr 29 kDa in einer reduzierenden SDS-Polyacrylamidgelelektrophorese auf aktivierten T-Lymphozyten erkennt.

7. Eine Hybridomzelle, die den monoklonalen Antikörper gemäß einem der Ansprüche 1 bis 6 erzeugt.

8. Verwendung eines monoklonalen Antikörpers, der ein ko-stimulierendes Molekül
a) mit der biologischen Aktivität der Ko-Stimulation von T-Zellen,
b) das auf aktivierten CD4⁺- und CD8⁺-T-Lymphozyten, aber nicht auf ruhenden oder aktivierten B-Zellen, Granulozyten, Monozyten, NK-Zellen oder dendritischen Zellen vorkommt,
c) das zwei Polypeptidketten aufweist, wobei das genannte Molekül ein Molekulargewicht von etwa 55 bis 60 kDa hat, bestimmt in einer nicht reduzierenden SDS-Polyacrylamidgelelektrophorese, und wobei die zwei Polypeptidketten des genannten Moleküls ein Molekulargewicht von etwa 27 kDa und etwa 29 kDa haben, gemessen in einer reduzierenden SDS-Polyacrylamidgelelektrophorese, und
d) enthaltend eine Aminosäuresequenz, die mindestens 80% Homologie mit der 199 Aminosäuren umfassenden Sequenz in Fig. 15 (SEQ ID NO:2) aufweist oder ein biologisch aktives Fragment davon, das ebenfalls eine ko-stimulierende Wirkung auf T-Zell-Lymphozyten zeigt, oder enthaltend die Aminosäuresequenz gemäß Fig. 15 (SEQ ID NO:2) oder ein biologisch aktives Fragment davon, das ebenfalls eine ko-stimulierende Wirkung auf T-Zell-Lymphozyten zeigt,
spezifisch erkennt, wobei der Antikörper 2-Signal-aktivierte, jedoch nicht ruhende T-Lymphozyten erkennt und wobei der Antikörper die biologische Aktivität des ko-stimulierenden Moleküls hemmt oder zusammen mit dem monoklonalen anti-CD3-Antikörper OKT3 menschliche T-Lymphozyten ko-stimuliert zur Herstellung von Arzneimitteln.

9. Verwendung gemäß Anspruch 8 zur Herstellung eines Arzneimittels zur Behandlung von Autoimmunkrankheiten, zur Verhinderung der Absto-βungsreakfionen bei Organtransplantationen und/oder zur Behandlung einer Dysregulation des Immunsystems.

10. Verwendung gemäß Anspruch 9, wobei die Autoimmunerktankung ausgewählt ist aus rheumatoide Arthritis, Morbus Bechterew, Sjögren-Syndrom und/oder Colitis ulcerosa.

11. Verwendung eines monoklonalen Antikörpers, der ein ko-stimulierendes Molekül
a) mit der biologischen Aktivität der Ko-Stimulation von T-Zellen,
b) das auf aktivierten CD4⁺- und CD8⁺-T-Lymphozyten, aber nicht auf ruhenden oder aktivierten B-Zellen, Granulozyten, Monozyten, NK-Zellen oder dendritischen Zellen vorkommt,
c) das zwei Polypeptidketten aufweist, wobei das genannte Molekül ein Molekulargewicht von etwa 55 bis 60 kDa hat, bestimmt in einer nicht reduzierenden SDS-Polyacrylamidgelelektrophorese, und wobei die zwei Polypeptidketten des genannten Moleküls ein Molekulargewicht von etwa 27 kDa und etwa 29 kDa haben, gemessen in einer reduzierenden SDS-Polyacrylamidgelelektrophorese, und
d) enthaltend eine Aminosäuresequenz, die mindestens 80% Homologie mit der 199 Aminosäuren umfassenden Sequenz in Fig. 15 (SEQ ID NO:2) aufweist oder ein biologisch aktives Fragment davon, das ebenfalls eine ko-stimulierende Wirkung auf T-Zell-Lymphozyten zeigt, oder enthaltend die Aminosäuresequenz gemäß Fig. 15 (SEQ ID NO:2) oder ein biologisch aktives Fragment davon, das ebenfalls eine ko-stimulierende Wirkung auf T-Zell-Lymphozyten zeigt,
spezifisch erkennt, wobei der Antikörper 2-Signal-aktivierte, jedoch nicht ruhende T-Lymphozyten erkennt, zur Herstellung eines Mittels zur Diagnose von Erkrankungen, an denen das Immunsystem beteiligt ist.

12. Verwendung gemäß einem der Ansprüche 8-11, wobei die T-Lymphozyten CD4⁺-T-Zellen sind.

13. Verwendung gemäß einem der Ansprüche 8-11, wobei die T-Lymphozyten CD8⁺-T-Zellen sind.

14. Verwendung gemäß einem der Ansprüche 8-13, wobei der Antikörper ein Antigen von ungefähr 55-60 kDa in einer nicht-reduzierenden SDS-Polyacrylamidgelelektrophorese auf aktivierten T-Lymphozyten erkennt.

15. Verwendung gemäß einem der Ansprüche 8-13, wobei der Antikörper ein Antigen von ungefähr 27 und ungefähr 29 kDa in einer reduzierenden SDS-Polyacrylamidgelelektrophorese auf aktivierten T-Lymphozyten erkennt.

16. Verwendung eines ko-stimulierenden Moleküls
a) mit der biologischen Aktivität der Ko-Stimulation von T-Zellen,
b) das auf aktivierten CD4⁺- und CD8⁺-T-Lymphozyten, aber nicht auf ruhenden oder aktivierten B-Zellen, Granulozyten, Monozyten, NK-Zellen oder dendritischen Zellen vorkommt,
c) das zwei Polypeptidketten aufweist, wobei das genannte Molekül ein Molekulargewicht von etwa 55 bis 60 kDa hat, bestimmt in einer nicht reduzierenden SDS-Polyacrylamidgelelektrophorese, und wobei die zwei Polypeptidketten des genannten Moleküls ein Molekulargewicht von etwa 27 kDa und etwa 29 kDa haben, gemessen in einer reduzierenden SDS-Polyacrylamidgelelektrophorese, und
d) enthaltend eine Aminosäuresequenz, die mindestens 80% Homologie mit der 199 Aminosäuren umfassenden Sequenz in Fig. 15 (SEQ ID NO:2) aufweist oder ein biologisch aktives Fragment davon, das ebenfalls eine ko-stimulierende Wirkung auf T-Zell-Lymphozyten zeigt, oder enthaltend die Aminosäuresequenz gemäß Fig. 15 (SEQ ID NO:2) oder ein biologisch aktives Fragment davon, das ebenfalls eine ko-stimulierende Wirkung auf T-Zell-Lymphozyten zeigt,
zur Herstellung von Arzneimitteln zur Behandlung von Krebserkrankungen, Aids, Asthmaerkrankungen und/oder chronischen viralen Erkrankungen wie HCV- oder HBV-Infektionen.

17. Verwendung von Zellen, enthaltend ein ko-stimulierendes Molekül
a) mit der biologischen Aktivität der Ko-Stimulation von T-Zellen,
b) das auf aktivierten CD4⁺- und CD8⁺-T-Lymphozyten, aber nicht auf ruhenden oder aktivierten B-Zellen, Granulozyten, Monozyten, NK-Zellen oder dendritischen Zellen vorkommt,
c) das zwei Polypeptidketten aufweist, wobei das genannte Molekül ein Molekulargewicht von etwa 55 bis 60 kDa hat, bestimmt in einer nicht reduzierenden SDS-Polyacrylamidgelelektrophorese, und wobei die zwei Polypeptidketten des genannten Moleküls ein Molekulargewicht von etwa 27 kDa und etwa 29 kDa haben, gemessen in einer reduzierenden SDS-Polyacrylamidgelelektrophorese, und
d) enthaltend eine Aminosäuresequenz, die mindestens 80% Homologie mit der 199 Aminosäuren umfassenden Sequenz in Fig. 15 (SEQ ID NO:2) aufweist oder ein biologisch aktives Fragment davon, das ebenfalls eine ko-stimulierende Wirkung auf T-Zell-Lymphozyten zeigt, oder enthaltend die Aminosäuresequenz gemäß Fig. 15 (SEQ ID NO:2) oder ein biologisch aktives Fragment davon, das ebenfalls eine ko-stimulierende Wirkung auf T-Zell-Lymphozyten zeigt,
zur Herstellung von Arzneimitteln.

18. Verwendung von Zellen gemäß Anspruch 17 zur Herstellung eines Arzneimittels zur Behandlung von Krebserkrankungen, Aids, Asthmaerkrankungen und/oder chronischen viralen Erkrankungen wie HCV- oder HBV-Infektionen.

19. Verwendung gemäß Anspruch 11, wobei zur Diagnose ein ELISA-Nachweis, eine Durchflusszytometrie, ein Western Blot, ein Radioimmun Assay, eine Nephelometrie oder eine histochemische Anfärbung verwendet wird.

20. Verwendung eines ko-stimulierenden Moleküls
a) mit der biologischen Aktivität der Ko-Stimulation von T-Zellen,
b) das auf aktivierten CD4⁺- und CD8⁺-T-Lymphozyten, aber nicht auf ruhenden oder aktivierten B-Zellen, Granulozyten, Monozyten, NK-Zellen oder dendritischen Zellen vorkommt,
c) das zwei Polypeptidketten aufweist, wobei das genannte Molekül ein Molekulargewicht von etwa 55 bis 60 kDa hat, bestimmt in einer nicht reduzierenden SDS-Polyacrylamidgelelektrophorese, und wobei die zwei Polypeptidketten des genannten Moleküls ein Molekulargewicht von etwa 27 kDa und etwa 29 kDa haben, gemessen in einer reduzierenden SDS-Polyacrylamidgelelektrophorese, und
d) enthaltend eine Aminosäuresequenz, die mindestens 80% Homologie mit der 199 Aminosäuren umfassenden Sequenz in Fig. 15 (SEQ ID NO:2) aufweist oder ein biologisch aktives Fragment davon, das ebenfalls eine ko-stimulierende Wirkung auf T-Zell-Lymphozyten zeigt, oder enthaltend die Aminosäuresequenz gemäß Fig. 15 (SEQ ID NO:2) oder ein biologisch aktives Fragment davon, das ebenfalls eine ko-stimulierende Wirkung auf T-Zell-Lymphozyten zeigt,
zur *in-vitro*-Herstellung von Antikörpern.

21. Verfahren zur Herstellung von monoklonalen Antikörpern, bei dem B-Zellen von Mäusen, die mit PMA und der Ca²⁺-Ionophore Ionomycin aktivierten humanen T-Lymphozyten immunisiert werden, mit einer Myelomzelllinie zu einem Antikörper-sezernierenden Hybridom fusioniert werden und die monoklonalen Antikörper in der Durchflusszytometrie auf 2-Signal-Molekül aktivierte gegen ruhende T-Zellen gereinigt und als Antikörper identifiziert werden, die ein ko-stimulierendes Molekül
a) mit der biologischen Aktivität der Ko-Stimulation von T-Zellen,
b) das auf aktivierten CD4⁺- und CD8⁺-T-Lymphozyten, aber nicht auf ruhenden oder aktivierten B-Zellen, Granulozyten, Monozyten, NK-Zellen oder dendritischen Zellen vorkommt,
c) das zwei Polypeptidketten aufweist, wobei das genannte Molekül ein Molekulargewicht von etwa 55 bis 60 kDa hat, bestimmt in einer nicht reduzierenden SDS-Polyacrylamidgelelektrophorese, und wobei die zwei Polypeptidketten des genannten Moleküls ein Molekulargewicht von etwa 27 kDa und etwa 29 kDa haben, gemessen in einer reduzierenden SDS-Polyacrylamidgelelektrophorese, und
d) enthaltend eine Aminosäuresequenz, die mindestens 80% Homologie mit der 199 Aminosäuren umfassenden Sequenz in Fig. 15 (SEQ ID NO:2) aufweist oder ein biologisch aktives Fragment davon, das ebenfalls eine ko-stimulierende Wirkung auf T-Zell-Lymphozyten zeigt, oder enthaltend die Aminosäuresequenz gemäß Fig. 15 (SEQ ID NO:2) oder ein biologisch aktives Fragment davon, das ebenfalls eine ko-stimulierende Wirkung auf T-Zell-Lymphozyten zeigt,
spezifisch erkennen.

22. Verfahren gemäß Anspruch 21, wobei der Antikörper T-Lymphozyten ko-stimuliert.

23. Verfahren gemäß Anspruch 21, wobei der Antikörper die biologische Aktivität des ko-stimulierenden Moleküls hemmt oder zusammen mit dem monoklonalen anti-CD3-Antikörper OKT3, humane T-Lymphozyten ko-stimuliert.

24. Verfahren gemäß Anspruch 22, wobei die T-Lymphozyten CD4⁺-T-Zellen sind.

25. Verfahren gemäß Anspruch 22, wobei die T-Lymphozyten CD8⁺-T-Zellen sind.

26. Verfahren gemäß Anspruch 21, wobei der Antikörper ein Antigen von ungefähr 55 bis 60 kDa in einer nicht-reduzierenden SDS-Polyacrylamidgelelektrophorese auf aktivierten Lymphozyten erkennt.

27. Verfahren gemäß Anspruch 21, wobei der Antikörper ein Antigen von ungefähr 27 und ungefähr 29 kDa in einer reduzierenden SDS-Polyacrylamidgelelektrophorese auf aktivierten T-Lymphozyten erkennt.

28. Ein Medikament, das einen Antikörper enthält, der ein humanes ko-stimulierendes Molekül
a) mit der biologischen Aktivität der Ko-Stimulation von T-Zellen,
b) das auf aktivierten CD4⁺- und CD8⁺-T-Lymphozyten, aber nicht auf ruhenden oder aktivierten B-Zellen, Granulozyten, Monozyten, NK-Zellen oder dendritischen Zellen vorkommt,
c) das zwei Polypeptidketten aufweist, wobei das genannte Molekül ein Molekulargewicht von etwa 55 bis 60 kDa hat, bestimmt in einer nicht reduzierenden SDS-Polyacrylamidgelelektrophorese, und wobei die zwei Polypeptidketten des genannten Moleküls ein Molekulargewicht von etwa 27 kDa und etwa 29 kDa haben, gemessen in einer reduzierenden SDS-Polyacrylamidgelelektrophorese, und
d) enthaltend eine Aminosäuresequenz, die mindestens 80% Homologie mit der 199 Aminosäuren umfassenden Sequenz in Fig. 15 (SEQ ID NO:2) aufweist oder ein biologisch aktives Fragment davon, das ebenfalls eine ko-stimulierende Wirkung auf T-Zell-Lymphozyten zeigt, oder enthaltend die Aminosäuresequenz gemäß Fig. 15 (SEQ ID NO:2) oder ein biologisch aktives Fragment davon, das ebenfalls eine ko-stimulierende Wirkung auf T-Zell-Lymphozyten zeigt,
spezifisch erkennt, wobei der Antikörper 2-Signal-aktivierte, jedoch nicht ruhende T-Lymphozyten erkennt und wobei der Antikörper die biologische Aktivität des ko-stimulierenden Moleküls hemmt oder zusammen mit dem monoklonalen anti-CD3-Antikörper OKT3 menschliche T-Lymphozyten ko-stimuliert.

## Claims

1. A mouse antibody which specifically recognizes a costimulating molecule
(a) having the biological activity of costimulation of T cells,
(b) which occurs on activated CD4+ and CD8+ T lymphocytes but not resting or activated B cells, granulocytes, monocytes, NK cells or dendritic cells,
(c) which has two polypeptide chains, the said molecule having a molecular weight of about 55 to 60 kDa determined in a nonreducing SDS polyacrylamide gel electrophoresis, and the two polypeptide chains of the said molecule having a molecular weight of about 27 kDa and about 29 kDa measured in a reducing SDS polyacrylamide gel electrophoresis, and
(d) comprising an amino-acid sequence which shows at least 80% homology with the sequence comprising 199 amino acid in Fig. 15 (SEQ ID NO:2), or a biologically active fragment thereof showing likewise a costimulatory effect on T-cell lymphocytes, or comprising the amino acid sequence shown in Fig. 15 (SEQ ID NO:2), or a biologically active fragment thereof showing likewise a costimulatory effect on T-cell lymphocytes;
whereas the antibody recognizes 2-signal-activated but not resting T lymphocytes and whereas the antibody inhibits the biological activity of the costimulating molecule or in conjunction with anti-CD3 monoclonal antibody OKT3, costimulates human T lymphocytes.

2. The antibody according to Claim 1, which is a monoclonal antibody.

3. The antibody according to Claim 2, whereas the T lymphocytes are CD4+ T cells.

4. The antibody according to Claim 2, whereas the T lymphocytes are CD8+ T cells.

5. The antibody according to any of the Claims 1 to 4, whereas the antibody recognizes an antigen of about 55 to 60 kDa in a non-reducing SDS-PAGE on activated T lymphocytes.

6. The antibody according to any of the Claims 1 to 4, whereas the antibody recognizes an antigen of about 27 and about 29 kDa in a reducing SDS-PAGE on activated T lymphocytes.

7. A hybridoma cell which generates the monoclonal antibody according to one of the Claims 1 to 6.

8. A use of a monoclonal antibody which specifically recognizes a costimulating molecule
(a) having the biological activity of costimulation of T cells,
(b) which occurs on activated CD4+ and CD8+ T lymphocytes but not resting or activated B cells, granulocytes, monocytes, NK cells or dendritic cells,
(c) which has two polypeptide chains, the said molecule having a molecular weight of about 55 to 60 kDa determined in a nonreducing SDS polyacrylamide gel electrophoresis, and the two polypeptide chains of the said molecule having a molecular weight of about 27 kDa and about 29 kDa measured in a reducing SDS polyacrylamide gel electrophoresis, and
(d) comprising an amino-acid sequence which shows at least 80% homology with the sequence comprising 199 amino acid in Fig. 15 (SEQ ID NO:2), or a biologically active fragment thereof showing likewise a costimulatory effect on T-cell lymphocytes, or comprising the amino acid sequence shown in Fig. 15 (SEQ ID NO:2), or a biologically active fragment thereof showing likewise a costimulatory effect on T-cell lymphocytes;
whereas the antibody recognizes 2-signal-activated but not resting T lymphocytes and whereas the antibody inhibits the biological activity of the costimulating molecule or in conjunction with anti-CD3 monoclonal antibody OKT3, costimulates human T lymphocytes, for the production of pharmaceuticals.

9. The use according to claim 8 for the production of a pharmaceutical for the treatment of autoimmune diseases, for the prevention of rejection reactions in organ transplants and/or for the treatment of dysregulation of the immune system.

10. The use according to Claim 9, wherein the autoimmune disease is selected from rheumatoid arthritis, ankylosing spondylitis, Sjögren's syndrome and/or ulcerative colitis.

11. A use of a monoclonal antibody which specifically recognizes a costimulating molecule
(a) having the biological activity of costimulation of T cells,
(b) which occurs on activated CD4+ and CD8+ T lymphocytes but not resting or activated B cells, granulocytes, monocytes, NK cells or dendritic cells,
(c) which has two polypeptide chains, the said molecule having a molecular weight of about 55 to 60 kDa determined in a nonreducing SDS polyacrylamide gel electrophoresis, and the two polypeptide chains of the said molecule having a molecular weight of about 27 kDa and about 29 kDa measured in a reducing SDS polyacrylamide gel electrophoresis, and
(d) comprising an amino-acid sequence which shows at least 80% homology with the sequence comprising 199 amino acid in Fig. 15 (SEQ ID NO:2), or a biologically active fragment thereof showing likewise a costimulatory effect on T-cell lymphocytes, or comprising the amino acid sequence shown in Fig. 15 (SEQ ID NO:2), or a biologically active fragment thereof showing likewise a costimulatory effect on T-cell lymphocytes;
whereas the antibody recognizes 2-signal-activated but not resting T lymphocytes, for the production of means for the diagnosis of disorders in which the immune system is involved.

12. The use of an antibody according to one of the Claims 8-11, whereas the T lymphocytes are CD4+ T cells.

13. The use of an antibody according to one of the Claims 8-11, whereas the T lymphocytes are CD8+ T cells.

14. The use according to one of the Claims 8-13, whereas the antibody recognizes an antigen of about 55 to 60 kDa in a non-reducing SDS-PAGE on activated T lymphocytes.

15. The use according to one of the Claims 8-13, whereas the antibody recognizes an antigen of about 27 and about 29 kDa in a reducing SDS-PAGE on activated T lymphocytes.

16. A use of a costimulating molecule
(a) having the biological activity of costimulation of T cells,
(b) which occurs on activated CD4+ and CD8+ T lymphocytes but not resting or activated B cells, granulocytes, monocytes, NK cells or dendritic cells,
(c) which has two polypeptide chains, the said molecule having a molecular weight of about 55 to 60 kDa determined in a nonreducing SDS polyacrylamide gel electrophoresis, and the two polypeptide chains of the said molecule having a molecular weight of about 27 kDa and about 29 kDa measured in a reducing SDS polyacrylamide gel electrophoresis, and
(d) comprising an amino-acid sequence which shows at least 80% homology with the sequence comprising 199 amino acid in Fig. 15 (SEQ ID NO:2), or a biologically active fragment thereof showing likewise a costimulatory effect on T-cell lymphocytes, or comprising the amino acid sequence shown in Fig. 15 (SEQ ID NO:2), or a biologically active fragment thereof showing likewise a costimulatory effect on T-cell lymphocytes;
for the production of pharmaceuticals for the treatment of cancers, Aids, asthmatic disorders and/or chronic viral diseases such as HCV or HBV infections.

17. A use of cells comprising a costimulating molecule
(a) having the biological activity of costimulation of T cells,
(b) which occurs on activated CD4+ and CD8+ T lymphocytes but not resting or activated B cells, granulocytes, monocytes, NK cells or dendritic cells,
(c) which has two polypeptide chains, the said molecule having a molecular weight of about 55 to 60 kDa determined in a nonreducing SDS polyacrylamide gel electrophoresis, and the two polypeptide chains of the said molecule having a molecular weight of about 27 kDa and about 29 kDa measured in a reducing SDS polyacrylamide gel electrophoresis, and
(d) omprising an amino-acid sequence which shows at least 80% homology with the sequence comprising 199 amino acid in Fig. 15 (SEQ ID NO:2), or a biologically active fragment thereof showing likewise a costimulatory effect on T-cell lymphocytes, or comprising the amino acid sequence shown in Fig. 15 (SEQ ID NO:2), or a biologically active fragment thereof showing likewise a costimulatory effect on T-cell lymphocytes;
for the production of pharmaceuticals.

18. The use of cells according to Claim 17 for the production of a pharmaceutical for the treatment of cancers, Aids, asthmatic disorders and/or chronic viral diseases such as HCV or HBV infections.

19. The use according to Claim 11, where an ELISA detection, flow cytometry, Western blot, radioimmunoassay, nephelometry or a histochemical staining is used for the diagnosis.

20. A use of a costimulating molecule
(a) having the biological activity of costimulation of T cells,
(b) which occurs on activated CD4+ and CD8+ T lymphocytes but not resting or activated B cells, granulocytes, monocytes, NK cells or dendritic cells,
(c) which has two polypeptide chains, the said molecule having a molecular weight of about 55 to 60 kDa determined in a nonreducing SDS polyacrylamide gel electrophoresis, and the two polypeptide chains of the said molecule having a molecular weight of about 27 kDa and about 29 kDa measured in a reducing SDS polyacrylamide gel electrophoresis, and
(d) comprising an amino-acid sequence which shows at least 80% homology with the sequence comprising 199 amino acid in Fig. 15 (SEQ ID NO:2), or a biologically active fragment thereof showing likewise a costimulatory effect on T-cell lymphocytes, or comprising the amino acid sequence shown in Fig. 15 (SEQ ID NO:2), or a biologically active fragment thereof showing likewise a costimulatory effect on T-cell lymphocytes;
for producing antibodies in vitro.

21. A method of producing monoclonal antibodies, wherein B cells of mice which are immunized with human T lymphocytes activated PMA and the Ca2+ ionophore ionomycin are fused with a myeloma cell line to give an antibody-secreting hybridoma, and the monoclonal antibodies are purified in flow cytometry for 2-signal molecule-activated against resting T cells and identified as antibodies which specifically recognize a costimulating molecule
(a) having the biological activity of costimulation of T cells,
(b) which occurs on activated CD4+ and CD8+ T lymphocytes but not resting or activated B cells, granulocytes, monocytes, NK cells or dendritic cells,
(c) which has two polypeptide chains, the said molecule having a molecular weight of about 55 to 60 kDa determined in a nonreducing SDS polyacrylamide gel electrophoresis, and the two polypeptide chains of the said molecule having a molecular weight of about 27 kDa and about 29 kDa measured in a reducing SDS polyacrylamide gel electrophoresis, and
(d) comprising an amino-acid sequence which shows at least 80% homology with the sequence comprising 199 amino acid in Fig. 15 (SEQ ID NO:2), or a biologically active fragment thereof showing likewise a costimulatory effect on T-cell lymphocytes, or comprising the amino acid sequence shown in Fig. 15 (SEQ ID NO:2), or a biologically active fragment thereof showing likewise a costimulatory effect on T-cell lymphocytes.

22. The method according to claim 21, whereas the antibody costimulates T lymphocytes.

23. The method according to Claim 21, whereas the antibody inhibits the biological activity of the costimulating molecule or in conjunction with anti-CD3 monoclonal antibody OKT3, costimulates human T lymphocytes.

24. The method according to Claim 22, whereas the T lymphocytes are CD4+ T cells.

25. The method according to Claim 22, whereas the T lymphocytes are CD8+ T cells.

26. The method according to Claim 22, whereas the antibody recognizes an antigen of about 55 to 60 kDa in a non-reducing SDS-PAGE on activated T lymphocytes.

27. The method according to Claims 21, whereas the antibody recognizes an antigen of about 27 and about 29 kDa in a reducing SDS-PAGE on activated T lymphocytes.

28. A medicament containing an antibody which specifically recognizes a costimulating molecule
(a) having the biological activity of costimulation of T cells,
(b) which occurs on activated CD4+ and CD8+ T lymphocytes but not resting or activated B cells, granulocytes, monocytes, NK cells or dendritic cells,
(c) which has two polypeptide chains, the said molecule having a molecular weight of about 55 to 60 kDa determined in a nonreducing SDS polyacrylamide gel electrophoresis, and the two polypeptide chains of the said molecule having a molecular weight of about 27 kDa and about 29 kDa measured in a reducing SDS polyacrylamide gel electrophoresis, and
(d) comprising an amino-acid sequence which shows at least 80% homology with the sequence comprising 199 amino acid in Fig. 15 (SEQ ID NO:2), or a biologically active fragment thereof showing likewise a costimulatory effect on T-cell lymphocytes, or comprising the amino acid sequence shown in Fig. 15 (SEQ ID NO:2), or a biologically active fragment thereof showing likewise a costimulatory effect on T-cell lymphocytes;
whereas the antibody recognizes 2-signal-activated but not resting T lymphocytes and whereas the antibody inhibits the biological activity of the costimulating molecule or in conjunction with anti-CD3 monoclonal antibody OKT3, costimulates human T lymphocytes.

## Revendications

1. Un anticorps de souris qui reconnaît spécifiquement une molécule de co-stimulation
(a) ayant l'activité biologique de co-stimulation des cellules T,
(b) qui apparaît sur des lymphocytes T CD4⁺ et CD8⁺ activés mais non sur des cellules B naïves ou activées, des granulocytes, des monocytes, des cellules NK ou des cellules dendritiques,
(c) qui présente deux chaînes polypeptidiques, ladite molécule ayant un poids moléculaire, déterminé dans une électrophorèse sur gel de polyacrylamide SDS non réductrice, d'environ 55 à 60 kDa, et les deux chaînes polypeptidiques de ladite molécule ayant un poids moléculaire, mesuré dans une électrophorèse sur gel de polyacrylamide SDS réductrice, d'environ 27 kDa et d'environ 29 kDa, et
(d) comprenant une séquence d'acides aminés qui montre une homologie d'au moins 80% avec la séquence comprenant 199 acides aminés de la figure 15 (SEQ ID No. 2) ou un fragment biologiquement actif de celle-ci montrant de même un effet de co-stimulation sur des lymphocytes T ou comprenant la séquence d'acides aminés montrée dans la figure 15 (SEQ ID No. 2) ou un fragment biologiquement actif de celle-ci montrant de même un effet de co-stimulation sur des lymphocytes T,
dans lequel l'anticorps reconnaît les lymphocytes T activés par un signal 2 mais non naïfs et dans lequel l'anticorps inhibe l'activité biologique de la molécule de co-stimulation ou bien en association avec l'anticorps monoclonal anti-CD3 OKT3, co-stimule des lymphocytes T humains.

2. L'anticorps selon la revendication 1, qui consiste en un anticorps monoclonal.

3. L'anticorps selon la revendication 2, dans lequel les lymphocytes T sont des cellules T CD4⁺.

4. L'anticorps selon la revendication 2, dans lequel les lymphocytes T sont des cellules T CD8⁺.

5. L'anticorps selon l'une quelconque des revendications 1 à 4, dans lequel l'anticorps reconnaît un antigène d'environ 55 à 60 kDa dans une SDS-PAGE non réductrice sur des lymphocytes T activés.

6. L'anticorps selon l'une quelconque des revendications 1 à 4, dans lequel l'anticorps reconnaît un antigène d'environ 27 et d'environ 29 kDa dans une SDS-PAGE réductrice sur des lymphocytes T activés.

7. Une cellule d'hybridome qui génère l'anticorps monoclonal selon l'une quelconque des revendications 1 à 6.

8. Une utilisation d'un anticorps monoclonal qui reconnaît spécifiquement une molécule de co-stimulation
(a) ayant l'activité biologique de co-stimulation des cellules T,
(b) qui apparaît sur des lymphocytes T CD4⁺ et CD8⁺ activés mais non sur des cellules B naïves ou activées, des granulocytes, des monocytes, des cellules NK ou des cellules dendritiques,
(c) qui présente deux chaînes polypeptidiques, ladite molécule ayant un poids moléculaire, déterminé dans une électrophorèse sur gel de polyacrylamide SDS non réductrice, d'environ 55 à 60 kDa, et les deux chaînes polypeptidiques de ladite molécule ayant un poids moléculaire, mesuré dans une électrophorèse sur gel de polyacrylamide SDS réductrice, d'environ 27 kDa et d'environ 29 kDa, et
(d) comprenant une séquence d'acides aminés qui montre une homologie d'au moins 80% avec la séquence comprenant 199 acides aminés de la figure 15 (SEQ ID No. 2) ou un fragment biologiquement actif de celle-ci montrant de même un effet de co-stimulation sur des lymphocytes T ou comprenant la séquence d'acides aminés montrée dans la figure 15 (SEQ ID No. 2) ou un fragment biologiquement actif de celle-ci montrant de même un effet de co-stimulation sur des lymphocytes T,
dans lequel l'anticorps reconnaît les lymphocytes T activés par un signal 2 mais non naïfs et dans lequel l'anticorps inhibe l'activité biologique de la molécule de co-stimulation ou bien en association avec l'anticorps monoclonal anti-CD3 OKT3, co-stimule des lymphocytes T humains, pour la production de produits pharmaceutiques.

9. L'utilisation selon la revendication 8, pour la production d'un produit pharmaceutique destiné au traitement de maladies autoimmunes, à la prévention de réactions de rejet de transplants d'organes et/ou au traitement d'une dysrégulation du système immunitaire.

10. L'utilisation selon la revendication 9, dans laquelle la maladie autoimmune est l'une de celle du groupe comprenant la polyarthrite rhumatoïde, la spondylite ankylosante, le syndrome de Sjögren et/ou une colite ulcérante.

11. Utilisation d'un anticorps monoclonal qui reconnaît spécifiquement une molécule de co-stimulation
(a) ayant l'activité biologique de co-stimulation des cellules T,
(b) qui apparaît sur des lymphocytes T CD4+ et CD8+ activés mais non sur des cellules B naïves ou activées, des granulocytes, des monocytes, des cellules NK ou des cellules dendritiques,
(c) qui présente deux chaînes polypeptidiques, ladite molécule ayant un poids moléculaire, déterminé dans une électrophorèse sur gel de polyacrylamide SDS non réductrice, d'environ 55 à 60 kDa, et les deux chaînes polypeptidiques de ladite molécule ayant un poids moléculaire, mesuré dans une électrophorèse sur gel de polyacrylamide SDS réductrice, d'environ 27 kDa et d'environ 29 kDa, et
(d) comprenant une séquence d'acides aminés qui montre une homologie d'au moins 80% avec la séquence comprenant 199 acides aminés de la figure 15 (SEQ ID No. 2) ou un fragment biologiquement actif de celle-ci montrant de même un effet de co-stimulation sur des lymphocytes T ou comprenant la séquence d'acides aminés montrée dans la figure 15 (SEQ ID No. 2) ou un fragment biologiquement actif de celle-ci montrant de même un effet de co-stimulation sur des lymphocytes T,
dans laquelle l'anticorps reconnaît les lymphocytes T activés par un signal 2 mais non naïfs dans la production de moyens pour le diagnostic de troubles dans lesquels le système immunitaire est impliqué.

12. L'utilisation d'un anticorps selon l'une quelconque des revendications 8 à 11, dans laquelle les lymphocytes T sont des cellules T CD4⁺.

13. L'utilisation d'un anticorps selon l'une quelconque des revendications 8 à 11, dans laquelle les lymphocytes T sont des cellules T CD8⁺.

14. L'utilisation selon l'une quelconque des revendications 8 à 13, dans laquelle l'anticorps reconnaît un antigène d'environ 55 à 60 kDa dans une SDS-PAGE non réductrice sur des lymphocytes T activés.

15. L'utilisation selon l'une quelconque des revendications 8 à 13, dans laquelle l'anticorps reconnaît un antigène d'environ 27 et d'environ 29 kDa dans une SDS-PAGE réductrice sur des lymphocytes T activés.

16. Une utilisation d'une molécule de co-stimulation
(a) ayant l'activité biologique de co-stimulation des cellules T,
(b) qui apparaît sur des lymphocytes T CD4+ et CD8+ activés mais non sur des cellules B naïves ou activées, des granulocytes, des monocytes, des cellules NK ou des cellules dendritiques,
(c) qui présente deux chaînes polypeptidiques, ladite molécule ayant un poids moléculaire, déterminé dans une électrophorèse sur gel de polyacrylamide SDS non réductrice, d'environ 55 à 60 kDa, et les deux chaînes polypeptidiques de ladite molécule ayant un poids moléculaire, mesuré dans une électrophorèse sur gel de polyacrylamide SDS réductrice, d'environ 27 kDa et d'environ 29 kDa, et
(d) comprenant une séquence d'acides aminés qui montre une homologie d'au moins 80% avec la séquence comprenant 199 acides aminés de la figure 15 (SEQ ID No. 2) ou un fragment biologiquement actif de celle-ci montrant de même un effet de co-stimulation sur des lymphocytes T ou comprenant la séquence d'acides aminés montrée dans la figure 15 (SEQ ID No. 2) ou un fragment biologiquement actif de celle-ci montrant de même un effet de co-stimulation sur des lymphocytes T,
pour la production de produits pharmaceutiques destinés au traitement de cancers, du SIDA, de troubles asthmatiques et/ou de maladies virales chroniques comme des infections à HCV ou HBV.

17. Une utilisation de cellules comprenant une molécule de co-stimulation
(a) ayant l'activité biologique de co-stimulation des cellules T,
(b) qui apparaît sur des lymphocytes T CD4+ et CD8+ activés mais non sur des cellules B naïves ou activées, des granulocytes, des monocytes, des cellules NK ou des cellules dendritiques,
(c) qui présente deux chaînes polypeptidiques, ladite molécule ayant un poids moléculaire, déterminé dans une électrophorèse sur gel de polyacrylamide SDS non réductrice, d'environ 55 à 60 kDa, et les deux chaînes polypeptidiques de ladite molécule ayant un poids moléculaire, mesuré dans une électrophorèse sur gel de polyacrylamide SDS réductrice, d'environ 27 kDa et d'environ 29 kDa, et
(d) comprenant une séquence d'acides aminés qui montre une homologie d'au moins 80% avec la séquence comprenant 199 acides aminés de la figure 15 (SEQ ID No. 2) ou un fragment biologiquement actif de celle-ci montrant de même un effet de co-stimulation sur des lymphocytes T ou comprenant la séquence d'acides aminés montrée dans la figure 15 (SEQ ID No. 2) ou un fragment biologiquement actif de celle-ci montrant de même un effet de co-stimulation sur des lymphocytes T,
pour la production de produits pharmaceutiques.

18. L'utilisation de cellules selon la revendication 17 pour la production d'un produit pharmaceutique destiné au traitement de cancers, du SIDA, de troubles asthmatiques et/ou de maladies virales chroniques comme des infections par HCV ou HBV.

19. L'utilisation selon la revendication 11, où une détection ELISA, une cytométrie en flux, un transfert de protéines Western (Western blot), un radioimmunoessai, une néphélométrie ou une coloration histochimique est utilisé pour le diagnostic.

20. L'utilisation d'une molécule de co-stimulation
(a) ayant l'activité biologique de co-stimulation des cellules T,
(b) qui apparaît sur des lymphocytes T CD4+ et CD8+ activés mais non sur des cellules B naïves ou activées, des granulocytes, des monocytes, des cellules NK ou des cellules dendritiques,
(c) qui présente deux chaînes polypeptidiques, ladite molécule ayant un poids moléculaire, déterminé dans une électrophorèse sur gel de polyacrylamide SDS non réductrice, d'environ 55 à 60 kDa, et les deux chaînes polypeptidiques de ladite molécule ayant un poids moléculaire, mesuré dans une électrophorèse sur gel de polyacrylamide SDS réductrice, d'environ 27 kDa et d'environ 29 kDa, et
(d) comprenant une séquence d'acides aminés qui montre une homologie d'au moins 80% avec la séquence comprenant 199 acides aminés de la figure 15 (SEQ ID No. 2) ou un fragment biologiquement actif de celle-ci montrant de même un effet de co-stimulation sur des lymphocytes T ou comprenant la séquence d'acides aminés montrée dans la figure 15 (SEQ ID No. 2) ou un fragment biologiquement actif de celle-ci montrant de même un effet de co-stimulation sur des lymphocytes T,
pour la production d'anticorps *in vitro.*

21. Un procédé de production d'anticorps monoclonaux, dans lequel des cellules B de souris qui sont immunisées avec un PMA activé par des lymphocytes T humains et l'ionomycine ionophore Ca²⁺ sont fusionnées avec une lignée cellulaire de myélome pour donner un hybridome sécrétant des anticorps, et les anticorps monoclonaux sont purifiés par cytométrie en flux pour séparer les cellules activées par une molécule de signal 2 des cellules T naïves, et identifiés en tant qu'anticorps qui reconnaissent spécifiquement une molécule de co-stimulation
(a) ayant l'activité biologique de co-stimulation des cellules T,
(b) qui apparaît sur des lymphocytes T CD4+ et CD8+ activés mais non sur des cellules B naïves ou activées, des granulocytes, des monocytes, des cellules NK ou des cellules dendritiques,
(c) qui présente deux chaînes polypeptidiques, ladite molécule ayant un poids moléculaire, déterminé dans une électrophorèse sur gel de polyacrylamide SDS non réductrice, d'environ 55 à 60 kDa, et les deux chaînes polypeptidiques de ladite molécule ayant un poids moléculaire, mesuré dans une électrophorèse sur gel de polyacrylamide SDS réductrice, d'environ 27 kDa et d'environ 29 kDa, et
(d) comprenant une séquence d'acides aminés qui montre une homologie d'au moins 80% avec la séquence comprenant 199 acides aminés de la figure 15 (SEQ ID No. 2) ou un fragment biologiquement actif de celle-ci montrant de même un effet de co-stimulation sur des lymphocytes T ou comprenant la séquence d'acides aminés montrée dans la figure 15 (SEQ ID No. 2) ou un fragment biologiquement actif de celle-ci montrant de même un effet de co-stimulation sur des lymphocytes T.

22. Le procédé selon la revendication 21, dans lequel l'anticorps co-stimule les lymphocytes T.

23. Le procédé selon la revendication 21, dans lequel l'anticorps inhibe l'activité biologique de la molécule de co-stimulation, ou bien en association avec l'anticorps monoclonal anti-CD3 OKT3, co-stimule des lymphocytes T humains.

24. Le procédé selon la revendication 22, dans lequel les lymphocytes T sont des cellules T CD4⁺.

25. Le procédé selon la revendication 22, dans lequel les lymphocytes T sont des cellules T CD8⁺.

26. Le procédé selon la revendication 22, dans lequel l'anticorps reconnaît un antigène d'environ 55 à 60 kDa dans une SDS-PAGE non réductrice sur des lymphocytes T activés.

27. Le procédé selon la revendication 21, dans lequel l'anticorps reconnaît un antigène d'environ 27 et environ 29 kDa dans une SDS-PAGE réductrice sur des lymphocytes T activés.

28. Médicament contenant un anticorps qui reconnaît spécifiquement une molécule de co-stimulation
(a) ayant l'activité biologique de co-stimulation des cellules T,
(b) qui apparaît sur des lymphocytes T CD4+ et CD8+ activés mais non sur des cellules B naïves ou activées, des granulocytes, des monocytes, des cellules NK ou des cellules dendritiques,
(c) qui présente deux chaînes polypeptidiques, ladite molécule ayant un poids moléculaire, déterminé dans une électrophorèse sur gel de polyacrylamide SDS non réductrice, d'environ 55 à 60 kDa, et les deux chaînes polypeptidiques de ladite molécule ayant un poids moléculaire, mesuré dans une électrophorèse sur gel de polyacrylamide SDS réductrice, d'environ 27 kDa et d'environ 29 kDa, et
(d) comprenant une séquence d'acides aminés qui montre une homologie d'au moins 80% avec la séquence comprenant 199 acides aminés de la figure 15 (SEQ ID No. 2) ou un fragment biologiquement actif de celle-ci montrant de même un effet de co-stimulation sur des lymphocytes T ou comprenant la séquence d'acides aminés montrée dans la figure 15 (SEQ ID No. 2) ou un fragment biologiquement actif de celle-ci montrant de même un effet de co-stimulation sur des lymphocytes T,
dans lequel l'anticorps reconnaît les lymphocytes T activés par un signal 2 mais non naïfs et dans lequel l'anticorps inhibe l'activité biologique de la molécule de co-stimulation, ou bien en association avec l'anticorps monoclonal anti-CD3 OKT3, co-stimule des lymphocytes T humains.
